# EUROPEAN PATENT APPLICATION

(11) **EP 4 635 525 A1**
(43) Date of publication of application: **22.10.2025**
(21) Application number: 23903926.6
(22) Date of filing: 11.12.2023
(51) Int. Cl.: A61L 27/26, A61L 27/18, A61L 27/24, A61L 27/58, A61L 27/54, A61L 27/56, C08B 37/08, C08L 5/08, C08L 67/04, C08L 89/00

(54) **COMPOSITION FOR BONE REGENERATION AND BLOCK-TYPE BONE-REGENERATION SCAFFOLD INCLUDING SAME**

(30) Priority: 12.12.2022 KR 20220172818; 26.09.2023 KR 20230129719
(71) Applicant: Kyungpook National University Hospital, Jung-gu Daegu 41944 (KR); Kyungpook National University Industry-Academic Cooperation Foundation, Daegu 41566 (KR)
(72) Inventor: OH, Chang-Wug, Daegu 42028 (KR); LIM, Jeongok, Gunwi-gun, Gyeongsangbuk-do 43134 (KR); PARK, Kyeong-Hyeon, Daegu 41944 (KR); LEE, Kyueui, Daegu 41566 (KR)
(74) Representative: Symbiosis IP Limited
(86) International application number: PCT/KR2023/020318
(87) International publication number: WO 2024/128723

(57) **Abstract**

The present invention relates to a composition for bone regeneration, comprising: a biodegradable synthetic polymer; a biocompatible polymer compound comprising catechol groups; a collagen; and a bioactive substance, and provides a bone-regeneration scaffold comprising the composition for bone regeneration.

## Description

### [Technical Field]

The present disclosure relates to a composition for bone regeneration and a block-type bone-regeneration scaffold comprising the same, and specifically relates to a multilayer three-dimensional bone-regeneration scaffold comprising a composition for bone regeneration as a biologic graft for bone formation and bone regeneration.

### [Background Art]

In general, bone has the ability to heal minor injuries on its own, but a large range of bone defects caused by traumatic injury, tumor resection, or congenital disease lack the capacity for spontaneous regeneration. Current therapeutic approaches to treat this significant bone loss involve the use of complex scaffolding biomaterials designed to fill the bond defect space and provide mechanical scaffold containing biologically functional molecules such as bone growth proteins comprising BMP-2, BMP-7, TGF-beta, PDGF, and stem cells for effective bone regeneration.

Common methods used to restore bone defect areas comprise autologous bone grafting, in which a portion of the patient's own bone is harvested from another area and grafted; allogeneic bone grafting, in which bone from another person is chemically treated and then grafted; and xenogeneic bone grafting, in which bone from a non-human animal is chemically treated and then grafted. Autologous bone grafting, which is generally known as the best grafting method, has the disadvantage of requiring a secondary surgery in the patient, difficulty in obtaining the required amount, and complications in the donor site for bone grafting. Allogeneic bone grafting does not require a secondary surgery; however, it has a potential risk of an immune response, and there is a risk that viruses such as hepatitis may be introduced into the patient, although the probability is low. Xenobiotic bone grafting also has the disadvantage of immune response issues and the possibility of problems such as mad cow disease. Further, in addition to the above, bone regeneration treatments using biological agent are very limited in the application of demineralized bone matrix, bone morphogenetic growth factors, platelet-rich plasma, etc., and are not able to produce a continuous and consistent bone formation effect. Treatment using synthetic bone agents has problems such as lack of bone tissue regeneration and bone formation ability due to its inability to conform to the three-dimensional shape of bone, long period of time for complete skeletal substitution, and difficulty in restoring and regenerating bone tissue due to incomplete substitution in fatal bone defects. Accordingly, there is a need for bone grafting materials that reliably provide sufficient amounts of bone, are not susceptible to disease transmission, have excellent biocompatibility to replace existing graft materials, and are properly absorbable and replacable with regenerated bone upon grafting.

Recently, complex bone regeneration therapeutics that simultaneously achieve osteoinduction and osteoconduction have been commercialized and used in a primitive way by mixing bone graft materials such as hydroxyapatite and raw materials such as BMP-2 on the spot at the surgical site. However, these methods are limited by the transient release of growth factors, a lack of continuous bone regeneration efficacy, and an associated risk of infection at the surgical site.

In addition, BMPs are known to be the best bioactive substances capable of inducing bone regeneration, but there are problems in that it is difficult to maximize the bone regeneration efficiency due to limitations in the application method, and it is difficult to precisely control the amount, resulting in excessive or inadequate amounts to maximize the efficiency of bone regeneration.

Under these conditions, there is a need to develop a new type of bone regeneration material or bone-regeneration scaffold that overcome the limitations of existing bone regeneration treatments, are easy to apply to patients by medical professionals in the operating room, and have three-dimensional shapes and performance that reflect the latest patient-specific characteristics.

### [Disclosure]

### [Technical Problem]

An object of the present disclosure is to provide a composition for bone regeneration comprising a biodegradable synthetic polymer; a biocompatible polymer compound comprising catechol groups; collagen; and a bioactive substance.

Another object of the present disclosure is to provide a bone-regeneration scaffold comprising the composition for bone regeneration as described above.

Still another object of the present disclosure is to provide a composition for use in the treatment of bone diseases, comprising the composition for bone regeneration as described above.

Still another object of the present disclosure is to provide a method for manufacturing the bone-regeneration scaffold as described above.

Still another object of the present disclosure is to provide a biologic graft comprising the bone-regeneration scaffold as described above.

Still another object of the present disclosure is to provide a bone graft material comprising the bone-regeneration scaffold as described above.

Still another object of the present disclosure is to provide a bone substitute comprising the bone-regeneration scaffold as described above.

Still another object of the present disclosure is to provide a sustained-release composition comprising: a biodegradable synthetic polymer; a biocompatible polymer compound comprising catechol groups; collagen; and a bioactive substance.

Still another object of the present disclosure is to provide a sustained-release scaffold comprising the sustained-release composition as described above.

Still another object of the present disclosure is to provide a drug delivery system comprising: the sustained-release composition.

Still another object of the present disclosure is to provide a biologic graft comprising the sustained-release scaffold as described above.

Still another object of the present disclosure is to provide a method for use in the treatment of bone diseases, comprising: grafting into a subject any one of the composition for bone regeneration; the bone-regeneration scaffold; and the composition for use in the treatment of bone diseases according to the present disclosure.

Still another object of the present disclosure is to provide the use of any one of the composition for bone regeneration, the bone-regeneration scaffold, and the composition for use in the treatment of bone diseases of the present disclosure for use in the prevention or treatment of bone diseases.

### [Technical Solution]

Terms used in the present application are only used to describe specific embodiments and are not intended to limit the present disclosure. Singular expressions include plural expressions unless the context clearly indicates otherwise.

Various modifications may be made to the embodiments described below. The embodiments described below are not intended to limit the present disclosure, and should be understood to include all modifications, equivalents, or alternatives thereto.

In the present specification, terms such as "comprise", "have", and the like are intended to designate the presence of features, steps, structures, or combinations thereof described in the specification and it should not be understood as precluding the possibility of the presence or addition of one or more other features, steps, structures, or combinations thereof.

Further, unless defined otherwise, all terms used herein, including technical or scientific terms, have the same meaning as commonly understood by one of ordinary skill in the art to which the present disclosure belongs. Terms such as those defined in commonly used dictionaries should be interpreted as having a meaning consistent with the meaning in the context of the related art, and unless explicitly defined in the present application, it is not to be construed in an idealized or overly formal sense.

### Composition for bone regeneration

The present disclosure provides a composition for bone regeneration comprising a biodegradable synthetic polymer; a biocompatible polymer compound comprising catechol groups; collagen; and a bioactive substance.

The "biodegradable synthetic polymer" refers to a polymer that is degraded in a living body and has bioabsorbability and biocompatibility. In the present disclosure, the biodegradable synthetic polymer supports the deposition, growth, and differentiation of bone cells. The scaffold comprising the biodegradable synthetic polymer must be physically stable, exhibit physiological activity, and degrade after tissue formation, while simultaneously the decomposition product must be non-toxic. In addition, the scaffold may have properties and characteristics that are easy to shape by 3D printing or molding.

The biodegradable synthetic polymer may comprise at least one of poly-lactic acid (PLA), poly-L-lactic acid (PLLA), poly-D-lactic acid (PDLA), poly-glycolic acid (PGA), polycaprolactone, and poly-lactic-co-glycolic acid (PLGA), and may preferably be at least one of poly-lactic acid (PLA), poly-L-lactic acid (PLLA), polycaprolactone, and poly-lactic-co-glycolic acid (PLGA). In an embodiment, poly-L-lactic acid (PLLA) is used as the biodegradable synthetic polymer.

The "biocompatible polymer compound comprising catechol groups" may comprise a natural polymer having high biocompatibility and adhesiveness that acts as a coating and cross-linking agent through conjugation with a bioactive substance. The biocompatible polymer compound comprising catechol groups may form a hydrogen bond with the biodegradable synthetic polymer and increase the binding force with a bioactive substance (-NH₂, -SH) by comprising catechol groups. Examples thereof may comprise natural polymers such as catechol, gelatin, alginate, fibrin, and chitosan.

In the present disclosure, the biocompatible polymer compound comprising catechol groups may comprise at least one selected from the group consisting of a gelatin-catechol compound, an alginate-catechol compound, a fibrin-catechol compound, a chitosan-catechol compound, and polydopamine. Preferably, the biocompatible polymer compound comprising catechol groups may be an alginate-catechol compound, a chitosan-catechol compound, a polydopamine, or a combination thereof, and may be, more preferably, an alginate-catechol compound. In an embodiment, the alginate-catechol is a synthetic compound synthesized by reacting alginate and dopamine hydrochloride.

In addition, the biocompatible polymer compound comprisng catechol groups may be a polymer compound represented by the following Chemical Formula 1:

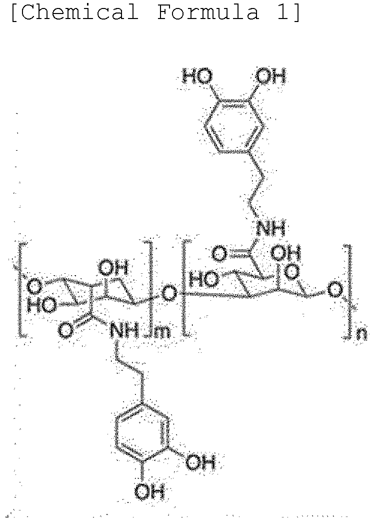

in Chemical Formula above, m and n are each independently any integer from 1 to 50.

The biocompatible polymer compound comprising catechol groups may act as a binder to fix the bioactive substance. The negatively charged carboxyl group and hydroxyl group of the biocompatible polymer compound comprising catechol groups may be conjugated by electrostatic interaction with the positively charged bioactive substance (specifically, BMP-2). In addition, a covalent bond may be formed between the catechol of the biocompatible polymer compound comprising catechol groups and the nucleophilic group of the bioactive substance (specifically, BMP-2).

As used herein, the term "collagen" refers to an extracellular matrix (ECM) protein that supplies bone with materials necessary for bone regeneration, which may have various types (collagen type I, IV, V, and VII, atelocollagen, etc.).

In the present disclosure, collagen may comprise collagen type I, and in an embodiment, collagen type I derived from porcine skin is used. The collagen type I may provide hydrophilicity to hydrophobic biodegradable synthetic polymers (e.g., PLLA) to increase a cell adhesion rate, and may increase the binding force with bioactive substances (e.g., BMP-2) by electrostatic attraction to control the release of BMP-2.

As used herein, the term "bioactive substance" refers to a substance capable of inducing a desired biological or pharmacological effect by promoting or inhibiting a physiological function in an animal or human body, for example, a biological substance or pharmacological substance that promotes healing ability in the process of bone tissue regeneration. The bioactive substances with osteogenic (bone formation) effects may participate in the healing of endochondral membranous fractures and enhance the effect of promoting bone growth.

Specifically, the bioactive substance may comprise a drug, protein, peptide, miRNA, siRNA, DNA, plasmid DNA, small molecules, antibody, virus, microorganism, somatic cell nucleus, organelle, mitochondria, enzyme, dietary supplement, vitamin, natural product, extract, growth factor, or cell, etc.

In an embodiment, the bioactive substance may comprise bone morphogenetic protein (BMP), platelet derived growth factor (PDGF), transforming growth factor beta (TGF-beta), basic fibroblast growth factor (bFGF), insulin-like growth factor 1 (IGF-1), lactoferrin, bisphosphonate, progenitor cells, bone marrow cells, epithelial cells, fibroblasts, osteoblasts, chondrocytes, cardiomyocytes, myocytes, hepatocytes, human-derived umbilical cord blood cells, mesenchymal stem cells, bone marrow-derived stem cells, periosteum-derived stem cells, vascular endothelial progenitor cells, embryonic stem cells, iPS-derived stem cells, adipose-derived stem cells, placental-derived stem cells, umbilical cord blood-derived stem cells, muscle-derived stem cells, induced pluripotent stem cells, fibroblasts, chondrocytes, osteoblasts, vascular endothelial cells, myoblasts, smooth muscle cells, hepatocytes, neural cells, cardiomyocytes, intervertebral disc cells, catechins or epigallocatechin gallate (EGCG).

Specifically, bone morphogenetic protein (BMP), a bioactive substance, is one of the homodimeric proteins derived from the bone matrix, and is known to have the function of regenerating bone by inducing bone differentiation of stem cells or progenitor cells and promoting osteoblast migration. Examples thereof comprise BMP-2, BMP-4, BMP-5, BMP-6, BMP-7, BMP-10, BMP-12, and BMP-13, etc. In addition, the BMP comprises natural and recombinant BMP, and the bone morphogenetic protein included in the bone formation (osteogenesis)-inducing composition according to an embodiment may be produced by a genetic recombination technique according to a method known in the art using prokaryotic and eukaryotic cell expression systems.

In the present disclosure, the bioactive substance may comprise BMP-1, BMP-2, BMP-3, BMP-4, BMP-5, BMP-6, and BMP-7 or a combination thereof, preferably BMP-2, BMP-7, or a combination thereof, and more preferably BMP-2. In an embodiment, rhBMP-2 is used as the bioactive substance.

The BMP-2 may induce osteogenic differentiation and osteogenesis of multipotent mesenchymal cells. The bioactive substances such as BMP-2 may enhance bone regeneration *in vivo,* but they have a short half-life and require an appropriate carrier to maintain proper bioactivity once administered. The BMP-2 has received FDA approval in the United States and Europe and is currently used in clinical use. However, when BMP-2 protein is used clinically, a relatively large amount is required due to its short half-life in the body, and excessive dosage causes a risk of tumor formation. Accordingly, the present disclosure implements a bone-regeneration scaffold comprising a bioactive substance as a carrier capable of improving the bone regeneration effect of BMP-2.

The composition for bone regeneration or the bone-regeneration scaffold comprising the composition for bone regeneration according to an embodiment may comprise a minimal amount of BMP-2 (e.g., BMP-2 at a concentration of 5 µg/mL or less) to exhibit maximum bone formation, bone regeneration or bone formation induction effect with a small amount. However, the amount of the BMP-2 may vary depending on the individual patient or the disease to be treated.

Further, in an embodiment, the bone-regeneration scaffold comprising the composition for bone regeneration may comprise bone marrow. The bone marrow may be impregnated into pores formed in the bone-regeneration scaffold of the present disclosure or may be adsorbed onto a surface of the pores. The bone-regeneration scaffold of the present disclosure may comprise the bone marrow in an amount of 1 ml or less, such that even a small amount is sufficient to achieve maximum bone formation, bone regeneration or bone formation induction effects.

The term "bone regeneration" as used herein may refer to all phenomena that treat, alleviate, or improve bone damage through processes such as proliferation of bone cells or differentiation of osteoblasts into bone cells to treat damaged bone tissue, and in particular, the bone regeneration may occur by promoting bone formation or bone differentiation, but the present disclosure is not limited thereto.

The term "osteogenesis (bone formation)" as used herein refers to a process by which bone is developed, which may comprise bone matrix formation by osteoblasts and its calcification, and comprises promotion of bone formation by healing of lost bone tissue.

Based on 100 parts by weight of the composition for bone-regeneration, the composition may comprise 85 to 95 parts by weight of the biodegradable synthetic polymer; 0.5 to 5 parts by weight of the biocompatible polymer compound comprising catechol groups; 4 to 10 parts by weight of collagen; and 0.05 to 0.5 parts by weight of the bioactive substance.

Further, based on 100 parts by weight of the composition for bone-regeneration, the composition may comprise 90 to 93 parts by weight of the biodegradable synthetic polymer; 1 to 3 parts by weight of the biocompatible polymer compound comprising catechol groups; 5 to 8 parts by weight of collagen; and 0.08 to 0.3 parts by weight of the bioactive substance.

In an embodiment, based on 100 parts by weight of the composition for bone-regeneration, the composition may comprise 91 parts by weight of the biodegradable synthetic polymer; 1.9 parts by weight of the biocompatible polymer compound comprising catechol groups; 7 parts by weight of collagen; and 0.1 parts by weight of the bioactive substance.

### Bone-regeneration scaffold comprising composition for bone regeneration

In another general aspect, the present disclosure provides a bone-regeneration scaffold comprising the composition for bone regeneration as described above.

The bone-regeneration scaffold may comprise a multilayer structure in which a biodegradable synthetic polymer; a biocompatible polymer compound layer comprising catechol groups; collagen; and a bioactive substance are alternately and repeatedly stacked in any order.

The definitions of the "biodegradable synthetic polymer", "biocompatible polymer compound comprising catechol groups", "collagen" and "bioactive substance" are as described above in the composition for bone regeneration.

The bone-regeneration scaffold may comprise a multilayer structure in which a biocompatible polymer compound layer comprising catechol groups; a collagen layer; and a bioactive substance layer are alternately and repeatedly stacked in any order, on a porous scaffold comprising a biodegradable synthetic polymer.

The expression "on a porous scaffold comprising a biodegradable synthetic polymer" may comprise one side, both sides, or the entire surface of the porous scaffold.

The bone-regeneration scaffold of the present disclosure may comprise all of the "biocompatible polymer compound layer comprising catechol groups; collagen layer; and bioactive substance layer" stacked on the porous scaffold comprising the biodegradable synthetic polymer, and the layers of the same material may be overlapped or sequentially present.

For example, "PLLA porous scaffold / a biocompatible polymer compound layer comprising catechol groups / a bioactive substance / collagen / a biocompatible polymer compound layer comprising catechol groups / a bioactive substance / collagen" may be sequentially stacked on the porous scaffold.

In another example, "a biocompatible polymer compound layer comprising catechol groups / a bioactive substance / collagen" may be sequentially stacked on the porous scaffold.

In another example, "a biocompatible polymer compound layer comprising catechol groups / collagen / a bioactive substance / a biocompatible polymer compound layer comprising catechol groups / collagen / a bioactive substance" may be sequentially stacked on the porous scaffold.

In another example, "a biocompatible polymer compound layer comprising catechol groups / collagen / collagen / a bioactive substance" may be sequentially stacked on the porous scaffold.

In another example, "a collagen layer / a biocompatible polymer compound layer comprising catechol groups / a bioactive substance / a collagen layer / a bioactive substance / collagen" may be sequentially stacked on the porous scaffold.

In the present disclosure, the bone-regeneration scaffold may have a multilayer structure in which a biocompatible polymer compound layer comprising catechol groups is stacked on a porous scaffold comprising a biodegradable synthetic polymer; a bioactive substance layer is stacked on the biocompatible polymer compound layer comprising catechol groups; and a collagen layer is stacked on the bioactive substance layer, wherein the biocompatible polymer compound layer comprising catechol groups/bioactive substance layer/collagen layer sequentially stacked on the porous scaffold is one unit, and the units are repeatedly stacked.

The units may be repeatedly stacked 2 to 4 times, and preferably 2 to 3 times.

In an embodiment, a unit comprising a biocompatible polymer compound layer comprising catechol groups, a bioactive substance layer, and a collagen layer may be stacked 1 to 3 times sequentially on a porous scaffold comprising a biodegradable synthetic polymer.

For example, the bone-regeneration scaffold in which the units are stacked twice may comprise a form in which a biocompatible polymer compound layer comprising catechol groups, a bioactive substance layer, a collagen layer, a biocompatible polymer compound layer comprising catechol groups, a bioactive substance layer, and a collagen layer are stacked sequentially on a porous scaffold comprising a biodegradable synthetic polymer.

In an embodiment, the bone-regeneration scaffold may comprise a form in which a biocompatible polymer compound layer comprising catechol groups, a bioactive substance layer, and a collagen layer are sequentially stacked on a porous scaffold comprising a biodegradable synthetic polymer.

In an embodiment, the bone-regeneration scaffold may comprise a form in which a biocompatible polymer compound layer comprising catechol groups, a collagen layer, a bioactive substance layer, a biocompatible polymer compound layer comprising catechol groups, a collagen layer, and a bioactive substance layer are sequentially stacked on a porous scaffold comprising a biodegradable synthetic polymer.

In an embodiment, the bone-regeneration scaffold may comprise a form in which a biocompatible polymer compound layer comprising catechol groups, a collagen layer, a collagen layer, and a bioactive substance layer are sequentially stacked on a porous scaffold comprising a biodegradable synthetic polymer.

In an embodiment, the bone-regeneration scaffold may comprise a form in which a collagen layer, a biocompatible polymer compound layer comprising catechol groups, a bioactive substance layer, a collagen layer, a bioactive substance layer, and a collagen layer are sequentially stacked on a porous scaffold comprising a biodegradable synthetic polymer.

The term "bone-regeneration scaffold" as used herein may be a biologic graft capable of replacing a part of a bone or bone tissue damaged in a living body and supplementing or replacing their functions, or a bone graft material capable of filling a space within bone tissue. In the present disclosure, the scaffold may have a block shape with thickness and three-dimensionality. In particular, the bone-regeneration scaffold may comprise a biodegradable polymer material, which may be completely degraded and disappear *in vivo* after being maintained until it sufficiently performs its function and role. The bone-regeneration scaffold does not necessarily have to match the shape of the bone damage site targeted for graft, and it is sufficient if it is shaped and processed to a similar shape, regardless of its shape. However, if necessary, the shape of the bone damage site targeted to the graft may be identified in advance, allowing for the scaffold to be manufactured using 3D printing or molding techniques to match that shape. The scaffold is a bioimplantable composite medical device that, once implanted in the body, performs its intended function, by not only maintaining strength to withstand repeated loads and momentary pressure, but also exhibiting biocompatibility or suitability.

The bone-regeneration scaffold may further comprise various materials to increase formability and physical properties.

The bone-regeneration scaffold of the present disclosure has a bone regeneration effect even without being grafted together with osteoblasts, and thus there is no concern about side effects such as immune responses upon grafting, and bone cell adhesion may be improved and bone differentiation may be promoted, thereby providing excellent effects as a bone-regeneration scaffold.

The term "3D printing" as used herein is a term that is commonly synonymous with rapid prototyping (RP) technology, and refers to a technology capable of producing an actual three-dimensional (3D) model in a short period of time by systematically layering materials one by one through 2D cross-sectioning of 3D image data.

In addition, the porous scaffold comprising the biodegradable synthetic polymer may have a lattice structure comprising pores.

The porous scaffold may be in the form of a block comprising pores.

In an embodiment, the porous scaffold may be a cylindrical shape comprising pores. However, the shape of the porous scaffold is not limited thereto and may be various shapes such as circular, oval, and polygonal shapes suitable for the graft site.

The porous scaffold may comprise pores having a diameter of 200 to 800 µm. Preferably, the porous scaffold may comprise pores having a diameter of 300 to 700 µm, more preferably 350 to 650 µm, even more preferably 380 to 600 µm, and still more preferably 400 to 450 µm.

Further, the porous scaffold may have a porosity of 50% to 90%, preferably 60% to 85%, more preferably 65% to 85%, even more preferably 70% to 85%, and still more preferably 80% to 85%.

In addition, the porous scaffold may have excellent inter-connectivity of 90% or more, and thus bone formation-involving and bone formation-inducing bioactive substances are able to move efficiently.

In an embodiment, the average diameter of the porous scaffold may be 0.5 to 10 mm, specifically, 1 to 9 mm, 2 to 8 mm, 3 to 7 mm, 4 to 6 mm, and more specifically, 5 mm. However, the range of the average diameter is not limited thereto and may be adjusted depending on the graft site.

In an embodiment, the length of the porous scaffold may be 0.5 to 20 mm, specifically, 1 to 18 mm, 2 to 17 mm, 3 to 16 mm, 5 to 15 mm, and more specifically, 15 mm. However, the range of the length is not limited thereto and may be adjusted depending on the graft site.

In the present disclosure, the "diameter of the porous scaffold" and the "length of the porous scaffold" are in a relationship that is perpendicular to each other, and the length of the bone-regeneration scaffold is also expressed as the thickness of the bone-regeneration scaffold.

In addition, the porous scaffold may have a strand thickness of the lattice forming the lattice structure of from 100 to 300 µm, preferably 120 to 280 µm, more preferably 140 to 260 µm, and even more preferably 150 to 200 µm, and in an embodiment, the lattice strand thickness may be 160 to 190 µm.

In addition, the density of the porous scaffold may be 10 to 30D (g/mm³), preferably 12 to 28D, more preferably 13 to 26D. In an embodiment, the density of the porous scaffold may be 15D, 20D, or 25D. A pore size of the porous scaffold may be adjusted to control the density, and the pore size may be adjusted as the density is controlled.

In an embodiment, the porous scaffold may have a cylindrical structure having a lattice structure regularly arranged in a lattice pattern with regularly arranged pores. A 0.5 to 2 mm-length (or thickness) portion at each end of the porous scaffold may have a higher density than the remaining portion of the porous scaffold, and may have a diameter that is 0.2 to 2 mm larger than the remaining portion.

For example, the 0.5 to 2 mm-length (or thickness) portion at each end may have a diameter of 6 mm, and the remaining portion may have a diameter of 5 mm. These structural characteristics are intended to prevent breakage of the porous scaffold and contribute to improving its physical stability.

Further, the bone-regeneration scaffold may be a lattice structure comprising pores.

The bone-regeneration scaffold may be in the form of a block comprising pores.

In an embodiment, the bone-regeneration scaffold may be a cylindrical shape comprising pores. However, the shape of the bone-regeneration scaffold is not limited thereto and may be various shapes such as circular and polygonal shapes suitable for the graft site.

The bone-regeneration scaffold may comprise pores having a diameter of 200 to 800 µm. Preferably, the bone-regeneration scaffold may comprise pores having a diameter of 300 to 700 µm, more preferably 350 to 650 µm, even more preferably 380 to 600 µm, and still more preferably 400 to 400 µm.

Further, the bone-regeneration scaffold may have a porosity of 50% to 90%, preferably 60% to 85%, more preferably 65% to 85%, even more preferably 70% to 85%, and still more preferably 80% to 85%.

In addition, the bone-regeneration scaffold may have excellent inter-connectivity of 90% or more, and thus bone formation-involving and bone formation-inducing bioactive substances are able to move efficiently.

In an embodiment, the average diameter of the bone-regeneration scaffold may be 0.5 to 10 mm, specifically, 1 to 9 mm, 2 to 8 mm, 3 to 7 mm, 4 to 6 mm, and more specifically, 5 mm. However, the range of the average diameter is not limited thereto and may be adjusted depending on the site where the bone-regeneration scaffold is grafted.

In an embodiment, the length of the bone-regeneration scaffold may be 0.5 to 20 mm, specifically, 1 to 18 mm, 2 to 17 mm, 3 to 16 mm, 5 to 15 mm, and more specifically, about 15 mm. However, the range of the length is not limited thereto and may be adjusted depending on the site where the bone-regeneration scaffold is grafted.

In the present disclosure, the "diameter of the bone-regeneration scaffold" and the "length of the bone-regeneration scaffold" are in a relationship that is perpendicular to each other, and the length of the bone-regeneration scaffold is also expressed as the thickness of the bone-regeneration scaffold.

In addition, the bone-regeneration scaffold may have a strand thickness of the lattice forming the lattice structure of from 100 to 300 µm, preferably 120 to 280 µm, more preferably 140 to 260 µm, and even more preferably 150 to 200 µm, and in an embodiment, the lattice strand thickness may be 160 to 190 µm.

In addition, the the bone-regeneration scaffold may comprise a bioactive substance at a concentration of 0.1 to 5 µg/mL, and specifically, may comprise a bioactive substance at a concentration of 0.1 to 4 µg/mL, 0.1 to 3 µg/mL, or 0.1 to 2 µg/mL. The bone-regeneration scaffold of the present disclosure comprises a bioactive substance at an optimal concentration capable of providing an excellent bone formation effect by releasing the bioactive substance at a constant rate for a long period of time while minimizing side effects that may occur when an excessive amount of the bioactive substance is administered.

The bone-regeneration scaffold may be used as a bone graft material for use in the treatment of bone diseases.

The bone disease may be growth retardation, osteopenia, bone fragility, osteonecrosis, fracture, osteoporosis caused by excessive osteoclast bone resorption, osteoporotic fracture, diabetic fracture, bone defect, nonunion, osteogenesis imperfecta, osteomalacia and fractures caused thereby, osteochondral interface defect, bone increase around graft correction, bone growth disorder, bone tumor, nonunion fracture, genetic bone growth defect, osteogenesis imperfecta, bone damage, traumatic joint damage, osteomalacia, rickets, osteitis fibrosa, aplastic bone disease, metabolic bone disease, renal osteodystrophy, etc.

The bone-regeneration scaffold is a biologic graft capable of being grafted into a bone defect site or a site requiring bone regeneration, and may be grafted and inserted through surgical and invasive procedures.

The bone-regeneration scaffold may be a sustained-release bone-regeneration scaffold that continuously releases the bioactive substance included in the bone-regeneration scaffold for a long period of time. The sustained-release characteristics of the bone-regeneration scaffold may be obtained by conjugation among the porous scaffold comprising the biodegradable synthetic polymer, the biocompatible polymer compound comprising catechol groups, the collagen, and the bioactive substance, included in the bone-regeneration scaffold. In particular, the release of the bioactive substance may be controlled by conjugation (e.g., electrostatic interaction or covalent bond) between the OH group contained in the biocompatible polymer compound comprising catechol groups (specifically, alginate-catechol) and the bioactive substance (Specifically, BMP-2), and thus the bioactive substance included in the bone-regeneration scaffold may be released consistently and continuously for a long period of time.

In addition, the bone-regeneration scaffold of the present disclosure may further comprise cells (e.g., bone marrow) that are included in a form that are impregnated into the pores of the bone-regeneration scaffold or adsorbed onto the pore surface. The bone-regeneration scaffold may have a synergistic effect on bone formation by comprising cells.

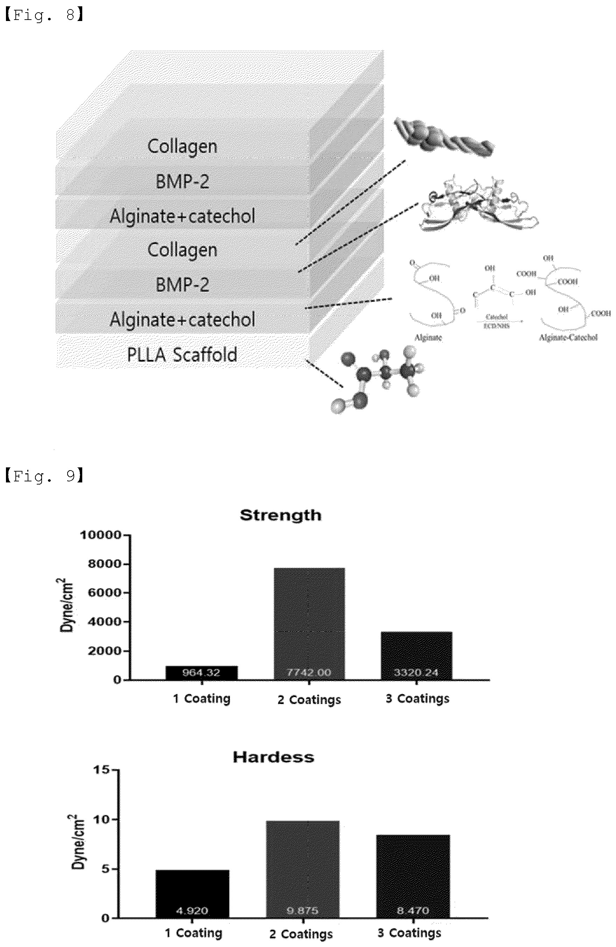

In an embodiment, BMP-collagen conjugation through coating the biocompatible polymer compound (Alginate-Catechol) comprising catechol groups on the PLLA porous scaffold is shown in FIG. 8.

### Composition for use in the treatment of bone diseases comprising composition for bone regeneration

In another general aspect, the present disclosure provides a composition for use in the treatment of bone diseases, comprising the composition for bone regeneration as described above.

The bone disease may be growth retardation, osteopenia, bone fragility, osteonecrosis, fracture, osteoporosis caused by excessive osteoclast bone resorption, osteoporotic fracture, diabetic fracture, bone defect, nonunion, osteogenesis imperfecta, osteomalacia and fractures caused thereby, osteochondral interface defect, bone increase around graft correction, bone growth disorder, bone tumor, nonunion fracture, genetic bone growth defect, osteogenesis imperfecta, bone damage, traumatic joint damage, osteomalacia, rickets, osteitis fibrosa, aplastic bone disease, metabolic bone disease, renal osteodystrophy, etc.

The term "treatment" means any act of improving or beneficially changing the symptoms of bone disease by administering the composition.

The composition may be used alone or in combination with methods using surgery, drug therapy, and biological response modifiers for the treatment of bone diseases.

The composition may be administered in combination with a bone-forming agent to improve the treatment of bone diseases, and may be administered in combination with drugs such as antibiotics, vitamins, etc., as adjuvants.

The terms "combination" or "combined" as used herein are not limited and may comprise fixed and non-fixed (e.g., free) forms (comprising kits) and uses, for example, simultaneous, sequential, or individual uses of components or elements.

The fracture may be treated by administering an effective amount of a therapeutic agent intended for the combination according to the present disclosure together.

As used herein, the term "effective amount" means the amount of an active ingredient or pharmaceutical composition that induces a biological or medical response in a tissue system, animal or human, as considered by a researcher, veterinarian, physician or other clinician, and comprises an amount that induces alleviation of symptoms of the disease or disorder. The effective amount and frequency of administration of the active ingredient of the present disclosure may vary depending on the desired effect. Therefore, the optimal dosage to be administered may be readily determined by those skilled in the art and may be adjusted according to a variety of factors, comprising the type of disease, the severity of the disease, the content of the active ingredient and other ingredients contained in the composition, the type of formulation, and the patient's age, weight, general health, gender and diet, time of administration, route of administration, and the excretion rate of the composition, duration of treatment, and drugs used simultaneously (in combination).

In the present disclosure, the composition may be administered by methods known in the art as bone graft materials or biologic inserts, and the composition may be administered locally to areas in need of defects or bone regeneration or bone formation by grafting using surgical and invasive procedures.

### Method for manufacturing bone-regeneration scaffold

In another general aspect, the present disclosure provides a method for manufacturing a bone-regeneration scaffold, the method comprising: (1) manufacturing a porous scaffold comprising a biodegradable synthetic polymer;
(2) coating any one of a biocompatible polymer compound comprising catechol groups, collagen, and a bioactive substance on the porous scaffold to form a first coating layer;
(3) coating, on the first coating layer, any one of a biocompatible polymer compound comprising catechol groups, collagen, and a bioactive substance on the porous scaffold to form a second coating layer; and
(4) coating, on the second coating layer, any one of a biocompatible polymer compound comprising catechol groups, collagen, and a bioactive substance on the porous scaffold to form a third coating layer,
wherein the first coating layer, the second coating layer, and the third coating layer are each independently repeated 1 to 3 times.

The definitions of the biodegradable synthetic polymer, the porous scaffold, the biocompatible polymer compound comprising catechol groups, the collagen, and the bioactive substance are as described above.

The porous scaffold comprising the biodegradable synthetic polymer may be formed by a 3D printing technique or a molding method.

The formation of the coating layer may be performed through a process of immersing and drying in a solution containing any one of the biocompatible polymer compound, collagen, and the bioactive substance.

Further, the formation of the coating layer comprising the biocompatible polymer compound comprising catechol groups and collagen may be performed at room temperature (15 to 25°C) or a temperature of 25 to 40°C, and the formation of the coating layer comprising the bioactive substance may be performed at a temperature of 10°C or lower, and preferably may be performed at a temperature of 5°C or lower.

In addition, the formation of the coating layer may be performed for 5 minutes to 72 hours, preferably for 30 minutes to 36 hours, and more preferably for 30 minutes to 30 hours, but the time for which the formation of the coating layer is performed is not limited thereto and may be appropriately adjusted depending on the temperature and amount. In an embodiment, the time may be about 1 hour, 2 hours, 3 hours, or about 24 hours.

After Step (4), a freeze-drying step (Step 5) may be further performed.

The freeze-drying may be performed for 40 to 100 hours, preferably for 40 to 80 hours.

After Step (5), a step of impregnating a bioactive substance into pores of the porous scaffold or adsorbing a bioactive substance onto a surface of the pores (Step 6) may be further performed.

### Sustained-release composition

In another aspect, the present disclosure provides a sustained-release composition comprising a biodegradable synthetic polymer; a biocompatible polymer compound comprising catechol groups; collagen; and a bioactive substance.

The descriptions of the "biodegradable synthetic polymer", "biocompatible polymer compound comprising catechol groups", "collagen" and "bioactive substance" in the sustained-release composition are as described above in the bone-regeneration scaffold.

Based on 100 parts by weight of the sustained-release composition, the composition may comprise 85 to 95 parts by weight of the biodegradable synthetic polymer; 0.5 to 5 parts by weight of the biocompatible polymer compound comprising catechol groups; 4 to 10 parts by weight of collagen; and 0.05 to 0.5 parts by weight of the bioactive substance.

Further, based on 100 parts by weight of the sustained-release composition, the composition may comprise 90 to 93 parts by weight of the biodegradable synthetic polymer; 1 to 3 parts by weight of the biocompatible polymer compound comprising catechol groups; 5 to 8 parts by weight of collagen; and 0.08 to 0.3 parts by weight of the bioactive substance.

In an embodiment, based on 100 parts by weight of the sustained-release composition, the composition may comprise 91 parts by weight of the biodegradable synthetic polymer; 1.9 parts by weight of the biocompatible polymer compound comprising catechol groups; 7 parts by weight of collagen; and 0.1 parts by weight of the bioactive substance.

The sustained-release composition has sustained-release characteristics that release the bioactive substance continuously and constantly for a long period of time.

Therefore, the sustained-release composition may continuously have a therapeutic effect suitable for the pharmacological effect of the bioactive substance included in the sustained-release composition.

### Sustained-release scaffold comprising sustained-release composition

In another general aspect, the present disclosure provides a sustained-release scaffold comprising the sustained-release composition for as described above.

The sustained-release scaffold may comprise a multilayer structure in which a biodegradable synthetic polymer; a biocompatible polymer compound comprising catechol groups; collagen; and a bioactive substance are alternately and repeatedly stacked in any order.

The definitions of the "biodegradable synthetic polymer", "biocompatible polymer compound comprising catechol groups", "collagen" and "bioactive substance" are as described above in the composition for bone regeneration.

The sustained-release scaffold may comprise a multilayer structure in which a biocompatible polymer compound layer comprising catechol groups; a collagen layer; and a bioactive substance layer are alternately and repeatedly stacked in any order, on a porous scaffold comprising a biodegradable synthetic polymer.

The expression "on a porous scaffold comprising a biodegradable synthetic polymer" may comprise one side, both sides, or the entire surface of the porous scaffold.

The sustained-release scaffold of the present disclosure may comprise all of the "biocompatible polymer compound layer comprising catechol groups; collagen layer; and bioactive substance layer" stacked on the porous scaffold comprising the biodegradable synthetic polymer, and the layers of the same material may be overlapped or sequentially present.

For example, "PLLA porous scaffold / a biocompatible polymer compound layer comprising catechol groups / a bioactive substance / collagen / a biocompatible polymer compound layer comprising catechol groups / a bioactive substance / collagen" may be sequentially stacked on the porous scaffold.

In another example, "a biocompatible polymer compound layer comprising catechol groups / a bioactive substance / collagen" may be sequentially stacked on the porous scaffold.

In another example, "a biocompatible polymer compound layer comprising catechol groups / collagen / a bioactive substance / a biocompatible polymer compound layer comprising catechol groups / collagen / a bioactive substance" may be sequentially stacked on the porous scaffold.

In another example, "a biocompatible polymer compound layer comprising catechol groups / collagen / collagen / a bioactive substance" may be sequentially stacked on the porous scaffold.

In another example, "a collagen layer / a biocompatible polymer compound layer comprising catechol groups / a bioactive substance / a collagen layer / a bioactive substance / collagen" may be sequentially stacked on the porous scaffold.

In the present disclosure, the sustained-release scaffold may have a multilayer structure in which a biocompatible polymer compound layer comprising catechol groups is stacked on a porous scaffold comprising a biodegradable synthetic polymer; a bioactive substance layer is stacked on the biocompatible polymer compound layer comprising catechol groups; and a collagen layer is stacked on the bioactive substance layer, wherein the biocompatible polymer compound layer comprising catechol groups / bioactive substance layer / collagen layer sequentially stacked on the porous scaffold is one unit, and the units are repeatedly stacked.

The units may be repeatedly stacked 2 to 4 times, and preferably 2 to 3 times.

In an embodiment, a unit comprising a biocompatible polymer compound layer comprising catechol groups, a bioactive substance layer, and a collagen layer may be stacked 1 to 3 times sequentially on a porous scaffold comprising a biodegradable synthetic polymer.

For example, the sustained-release scaffold in which the units are stacked twice may comprise a form in which a biocompatible polymer compound layer comprising catechol groups, a bioactive substance layer, a collagen layer, a biocompatible polymer compound layer comprising catechol groups, a bioactive substance layer, and a collagen layer are stacked sequentially on a porous scaffold comprising a biodegradable synthetic polymer.

In an embodiment, the sustained-release scaffold may comprise a form in which a biocompatible polymer compound layer comprising catechol groups, a bioactive substance layer, and a collagen layer are sequentially stacked on a porous scaffold comprising a biodegradable synthetic polymer.

In an embodiment, the sustained-release scaffold may comprise a form in which a biocompatible polymer compound layer comprising catechol groups, a collagen layer, a bioactive substance layer, a biocompatible polymer compound layer comprising catechol groups, a collagen layer, and a bioactive substance layer are sequentially stacked on a porous scaffold comprising a biodegradable synthetic polymer.

In an embodiment, the sustained-release scaffold may comprise a form in which a biocompatible polymer compound layer comprising catechol groups, a collagen layer, a collagen layer, and a bioactive substance layer are sequentially stacked on a porous scaffold comprising a biodegradable synthetic polymer.

In an embodiment, the sustained-release scaffold may comprise a form in which a collagen layer, a biocompatible polymer compound layer comprising catechol groups, a bioactive substance layer, a collagen layer, a bioactive substance layer, and a collagen layer are sequentially stacked on a porous scaffold comprising a biodegradable synthetic polymer.

The term "sustained-release scaffold" as used herein may be a biologic graft capable of replacing a part of a bone or bone tissue damaged in a living body and supplementing or replacing their functions, or a graft material capable of filling a space within bone tissue. In the present disclosure, the scaffold may have a block shape with thickness and three-dimensionality. In particular, the sustained-release scaffold may comprise a biodegradable polymer material, which may be completely degraded and disappear *in vivo* after being maintained until it sufficiently performs its function and role. The sustained-release scaffold does not necessarily have to match the shape of the damage site for graft, and it is sufficient if it is shaped and processed to a similar shape, regardless of its shape. However, if necessary, the shape of the bone damage site targeted to the graft may be identified in advance, allowing for the scaffold to be manufactured using 3D printing or molding techniques to match that shape. The scaffold is a bioimplantable composite medical device that, once implanted in the body, performs its intended function, by not only maintaining strength to withstand repeated loads and momentary pressure, but also exhibiting biocompatibility or suitability. The sustained-release scaffold may have the continuous and sustained release effect of the bioactive substance or drug impregnated or attached to the scaffold, thereby prolonging its pharmacological effect of the bioactive substance or drug for a long period of time.

The sustained-release scaffold may further comprise various materials to increase formability and physical properties.

The term "3D printing" as used herein is a term that is commonly synonymous with rapid prototyping (RP) technology, and refers to a technology capable of producing an actual three-dimensional (3D) model in a short period of time by systematically layering materials one by one through 2D cross-sectioning of 3D image data.

In addition, the porous scaffold comprising the biodegradable synthetic polymer may have a lattice structure comprising pores.

The porous scaffold may be in the form of a block comprising pores.

In an embodiment, the porous scaffold may be a cylindrical shape comprising pores. However, the shape of the porous scaffold is not limited thereto and may be various shapes such as circular, oval, and polygonal shapes suitable for the graft site.

The porous scaffold may comprise pores having a diameter of 200 to 800 µm. Preferably, the porous scaffold may comprise pores having a diameter of 300 to 700 µm, more preferably 350 to 650 µm, even more preferably 380 to 600 µm, and still more preferably 400 to 450 µm.

Further, the porous scaffold may have a porosity of 50% to 90%, preferably 60% to 85%, more preferably 65% to 85%, even more preferably 70% to 85%, and still more preferably 80% to 85%.

In addition, the porous scaffold may have excellent inter-connectivity of 90% or more, and thus bioactive substances are able to move efficiently.

In an embodiment, the average diameter of the porous scaffold may be 0.5 to 10 mm, specifically, 1 to 9 mm, 2 to 8 mm, 3 to 7 mm, 4 to 6 mm, and more specifically, 5 mm. However, the range of the average diameter is not limited thereto and may be adjusted depending on the graft site.

In an embodiment, the length of the porous scaffold may be 0.5 to 20 mm, specifically, 1 to 18 mm, 2 to 17 mm, 3 to 16 mm, 5 to 15 mm, and more specifically, 15 mm. However, the range of the average diameter is not limited thereto and may be adjusted depending on the graft site.

In the present disclosure, the "diameter of the porous scaffold" and the "length of the porous scaffold" are in a relationship that is perpendicular to each other, and the length of the sustained-release scaffold is also expressed as the thickness of the sustained-release scaffold.

In addition, the porous scaffold may have a strand thickness of the lattice forming the lattice structure of from 100 to 300 µm, preferably 120 to 280 µm, more preferably 140 to 260 µm, and even more preferably 150 to 200 µm, and in an embodiment, the lattice strand thickness may be 160 to 190 µm.

In addition, the density of the porous scaffold may be 10 to 30D (g/mm³), preferably 12 to 28D, more preferably 13 to 26D. In an embodiment, the density of the porous scaffold may be 15D, 20D, or 25D. A pore size of the porous scaffold may be adjusted to control the density, and the pore size may be adjusted as the density is controlled.

In an embodiment, the porous scaffold may have a cylindrical structure having a lattice structure regularly arranged in a lattice pattern with regularly arranged pores. A 0.5 to 2 mm-length (or thickness) portion at each end of the porous scaffold may have a higher density than the remaining portion of the porous scaffold, and may have a diameter that is 0.2 to 2 mm larger than the remaining portion.

For example, the 0.5 to 2 mm-length (or thickness) portion at each end may have a diameter of 6 mm, and the remaining portion may have a diameter of 5 mm. These structural characteristics are intended to prevent breakage of the porous scaffold and contribute to improving its physical stability.

Further, the sustained-release scaffold may be a lattice structure comprising pores.

The sustained-release scaffold may be in the form of a block comprising pores.

In an embodiment, the sustained-release scaffold may be a cylindrical shape comprising pores. However, the shape of the sustained-release scaffold is not limited thereto and may be various shapes such as circular and polygonal shapes suitable for the graft site.

The sustained-release scaffold may comprise pores having a diameter of 200 to 800 µm. Preferably, the sustained-release scaffold may comprise pores having a diameter of 300 to 700 µm, more preferably 350 to 650 µm, even more preferably 380 to 600 µm, and still more preferably 400 to 450 µm.

Further, the sustained-release scaffold may have a porosity of 50% to 90%, preferably 60% to 85%, more preferably 65% to 85%, even more preferably 70% to 85%, and still more preferably 80% to 85%.

In addition, the sustained-release scaffold may have excellent inter-connectivity of 90% or more, and thus bioactive substances having physiological or pharmaceutical therapeutic effects are able to move efficiently.

In an embodiment, the average diameter of the sustained-release scaffold may be 0.5 to 10 mm, specifically, 1 to 9 mm, 2 to 8 mm, 3 to 7 mm, 4 to 6 mm, and more specifically, about 5 mm. However, the range of the average diameter is not limited thereto and may be adjusted depending on the site where the sustained-release scaffold is grafted.

In an embodiment, the length of the sustained-release scaffold may be 0.5 to 20 mm, specifically, 1 to 18 mm, 2 to 17 mm, 3 to 16 mm, 5 to 15 mm, and more specifically, about 15 mm. However, the range of the length is not limited thereto and may be adjusted depending on the site where the sustained-release scaffold is grafted.

In the present disclosure, the "diameter of the sustained-release scaffold" and the "length of the sustained-release scaffold" are in a relationship that is perpendicular to each other, and the length of the sustained-release scaffold is also expressed as the thickness of the sustained-release scaffold.

In addition, the sustained-release scaffold may have a strand thickness of the lattice forming the lattice structure of from 100 to 300 µm, preferably 120 to 280 µm, more preferably 140 to 260 µm, and even more preferably 150 to 200 µm, and in an embodiment, the lattice strand thickness may be 160 to 190 µm.

In addition, the sustained-release scaffold may comprise a bioactive substance at a concentration of 0.1 to 5 µg/mL, and specifically, may comprise a bioactive substance at a concentration of 0.1 to 4 µg/mL, 0.1 to 3 µg/mL, or 0.1 to 2 µg/mL. The sustained-release scaffold of the present disclosure comprises a bioactive substance at an optimal concentration capable of providing an excellent biological or pharmaceutical therapeutic effect by releasing the bioactive substance at a constant rate for a long period of time while minimizing side effects that may occur when an excessive amount of the bioactive substance is administered.

The sustained-release scaffold may be a scaffold that continuously releases the bioactive substance included in the sustained-release scaffold for a long period of time. The sustained-release characteristics of the sustained-release scaffold may be obtained by conjugation among the porous scaffold comprising the biodegradable synthetic polymer, the biocompatible polymer compound comprising catechol groups, the collagen, and the bioactive substance, included in the sustained-release scaffold. In particular, the release of the bioactive substance may be controlled by conjugation (e.g., electrostatic interaction or covalent bond) between the OH group contained in the biocompatible polymer compound comprising catechol groups (specifically, alginate-catechol) and the bioactive substance, and thus the bioactive substance included in the sustained-release scaffold may be released consistently and continuously for a long period of time.

In another general aspect, the present disclosure provides a biologic graft comprising the sustained-release scaffold as described above.

### Sustained-release drug delivery system

In another aspect, the present disclosure provides a sustained-release drug delivery system comprising a biodegradable synthetic polymer; a biocompatible polymer compound comprising catechol groups; collagen; and a bioactive substance.

The descriptions of the "biodegradable synthetic polymer", "biocompatible polymer compound comprising catechol groups", "collagen" and "bioactive substance" in the sustained-release drug delivery system are as described above in the bone-regeneration scaffold.

Based on 100 parts by weight of the sustained-release drug delivery system, the system may comprise 85 to 95 parts by weight of the biodegradable synthetic polymer; 0.5 to 5 parts by weight of the biocompatible polymer compound comprising catechol groups; 4 to 10 parts by weight of collagen; and 0.05 to 0.5 parts by weight of the bioactive substance.

Further, based on 100 parts by weight of the sustained-release drug delivery system, the system may comprise 90 to 93 parts by weight of the biodegradable synthetic polymer; 1 to 3 parts by weight of the biocompatible polymer compound comprising catechol groups; 5 to 8 parts by weight of collagen; and 0.08 to 0.3 parts by weight of the bioactive substance.

In an embodiment, based on 100 parts by weight of the sustained-release drug delivery system, the system may comprise 91 parts by weight of the biodegradable synthetic polymer; 1.9 parts by weight of the biocompatible polymer compound comprising catechol groups; 7 parts by weight of collagen; and 0.1 parts by weight of the bioactive substance.

The sustained-release drug delivery system is effective for sustained-release that releases the bioactive substance at a constant rate for a long period of time. Therefore, the sustained-release drug delivery system may continuously have a therapeutic effect suitable for the pharmacological effect of the bioactive substance included therein.

### Method for use in the treatment of bone diseases of composition for bone regeneration, bone-regeneration scaffold, and composition for use in the treatment of bone disease, and use thereof

The present disclosure provides a method for use in the treatment of bone diseases, comprising grafting in a subject any one of the composition for bone regeneration, the bone-regeneration scaffold, and the composition for use in the treatment of bone disease according to the present disclosure.

As used herein, the term "subject" comprises an animal or human whose symptoms may be ameliorated by the grafting of the composition for bone regeneration, the bone-regeneration scaffold, and the composition for use in the treatment of bone disease according to the present disclosure. By administering to a subject the composition for bone regeneration, the bone-regeneration scaffold, and the composition for use in the treatment of bone disease according to the present disclosure, bone diseases may be effectively treated.

The grafting may be performed locally at a site requiring bone regeneration, bone restoration, or bone disease treatment through surgical or invasive surgery, using the composition for bone regeneration, the bone-regeneration scaffold, and the composition for use in the treatment of bone diseases according to the present disclosure.

Further, the present disclosure provides use in preventing or treating bone diseases of any one of the composition for bone regeneration, the bone-regeneration scaffold, the composition for use in the treatment of bone diseases, and a method for manufacturing a bone-regeneration scaffold according to the present disclosure.

The descriptions of "the composition for bone regeneration", "the bone-regeneration scaffold", "the composition for use in the treatment of bone diseases", and "bone diseases" in the method for use in the treatment of bone diseases of the composition for bone regeneration, the bone-regeneration scaffold, and the composition for use in the treatment of bone disease and the use thereof are as described above.

### [Advantageous Effects]

The composition for bone regeneration of the present disclosure and the bone-regeneration scaffold comprising the composition for bone regeneration may have excellent mechanical properties capable of being maintained in a living body for a long period of time, and control the release rate of a bioactive substance to enable long-term sustained release for continuous and constant release over a long period of time, thereby minimizing side effects caused by mass release of the bioactive substance and maximizing the bone regeneration effect.

In addition, the bone-regeneration scaffold of the present disclosure may be easily manufactured as a customized graft material through 3D printing or molding to fit the shape and size of the bone loss site (graft site), and may provide an economical medical product for bone regeneration by using an appropriate amount of a bioactive substance.

### [Description of Drawings]

FIG. 1 shows the reaction scheme of the alginate-catechol compound of Example 1, which is a biocompatible polymer compound containing catechol of the present disclosure.
FIG. 2 shows the NMR analysis results of the alginate-catechol compound of Example 1.
FIG. 3 shows the results of a degree of substitution (DOS) analysis of the catechol in the alginate-catechol compound of Example 1.
FIG. 4 shows an SEM image of a PLLA porous scaffold according to Example 2-1.
FIG. 5 shows an SEM image of a PLLA porous scaffold according to Example 2-6.
FIG. 6 shows a Microscope image of the PLLA porous scaffold according to Example 2-6.
FIG. 7 is a schematic diagram showing the process flow chart of the bone-regeneration scaffold according to Example 3-1 and the binding relationship between the coating layers.
FIG. 8 is a schematic diagram showing the bone-regeneration scaffold according to Example 3-1.
FIG. 9 is a graph showing the analysis results of the compressive strength and hardness according to the number of coatings.
FIG. 10 shows the analysis results of the release behavior of rhBMP-2 of the bone-regeneration scaffold according to Example 3-1.
FIG. 11 shows the analysis results of the release behavior of rhBMP-2 of the bone-regeneration scaffold according to Example 3-2.
FIG. 12 shows the analysis results of the release behavior of rhBMP-2 of the bone-regeneration scaffold according to Example 3-3.
FIG. 13 shows the analysis results of the release behavior of rhBMP-2 of the bone-regeneration scaffold according to Example 3-4.
FIG. 14 shows the analysis results of the release behavior of rhBMP-2 of the bone-regeneration scaffold according to Example 3-5.
FIG. 15 shows the analysis results of the release behavior of rhBMP-2 of the bone-regeneration scaffolds according to Example 3-6 to 3-9.
FIG. 16 shows the results of ALP activity analysis to confirm osteogenic differentiation and bioactivity of BMP-2.
FIG. 17 shows the results of Micro-CT image analysis after grafting of the bone-regeneration scaffold of the present disclosure in a calvarial defect mouse model.
FIG. 18 shows the results of histological evaluation after grafting of the bone-regeneration scaffold of the present disclosure in a calvarial defect mouse model.
FIG. 19 shows the process of creating a rabbit long-bone defect model and applying a sample.
FIG. 20 shows images of the graft site visually confirmed after grafting in the rabbit long-bone defect model.
FIG. 21 shows X-ray analysis results at 0 weeks and 8 weeks after grafting of the bone-regeneration scaffold of the present disclosure in the rabbit long-bone defect model.
FIG. 22 shows CT analysis results at 0 and 8 weeks after grafting of the bone-regeneration scaffold of the present disclosure in the rabbit long-bone defect model.
FIG. 23 shows Micro-CT analysis results of the proximal and cross-sectional areas at 8 weeks after grafting of the bone-regeneration scaffold of the present disclosure in the rabbit long-bone defect model.
FIG. 24 is a graph showing the results of bone mineral density (BMD), bone volume (BV), bone volume / tissue volume (BV/TV), and bone surface (BS) analysis at 8 weeks after grafting of the bone-regeneration scaffold of the present disclosure in the rabbit long-bone defect model.
FIG. 25 shows X-ray analysis results at 0 weeks and 8 weeks after grafting of the bone marrow-containing bone-regeneration scaffold of the present disclosure in the rabbit long-bone defect model.
FIG. 26 is a graph showing the results of bone mineral density (BMD), bone volume (BV), bone volume / tissue volume (BV/TV), and bone surface (BS) analysis at 8 weeks after grafting of the bone marrow-containing bone-regeneration scaffold of the present disclosure in the rabbit long-bone defect model.
FIG. 27 shows the histologic analysis results after grafting of the bone-regeneration scaffold and the bone marrow-containing bone-regeneration scaffold of the present disclosure in the rabbit long-bone defect model.
FIG. 28 shows the new bone analysis results after grafting of the bone-regeneration scaffold and the bone marrow-containing bone-regeneration scaffold of the present disclosure in the rabbit long-bone defect model.

### [Best Mode]

Hereinafter, embodiments of the present disclosure will be described in detail so that those skilled in the art are able to easily practice the present disclosure. However, the present disclosure may be implemented in various different forms and is not limited to Examples described herein.

Reagents and solvents described below were purchased from Sigma-Aldrich and Alfa Aesar unless otherwise specified.

### <Examples>

### Example 1. Synthesis of alginate-catechol (Alg + Ca) compound

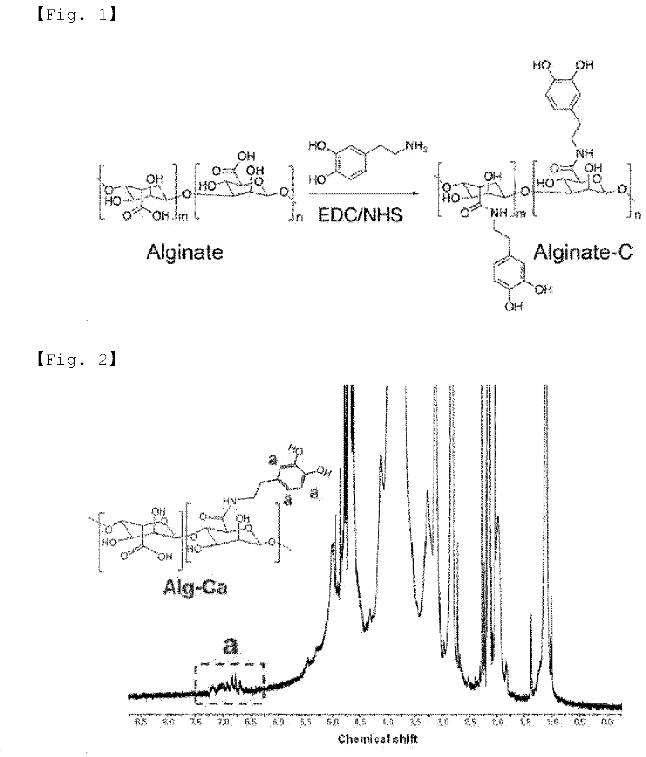

Alginate-catechol (Alg + Ca) was synthesized using the EDC (1-ethyl-3-(3'-dimethylaminopropyl)carbodiimide) / NHS (N-hydroxysuccinimide) coupling reaction (FIG. 1).

Alginate (1 g, ~5 mmol) was dissolved in 100 mL of 0.1 M MES buffer (pH 5.2) to prepare an alginate solution, and EDC-HCl (5 mmol) and NHS (5 mmol) were added dropwise to the prepared alginate solution to prepare an alginate / EDC / NHS solution. After bubbling with nitrogen for 5 minutes, dopamine hydrochloride (5 mmol) dissolved in 3 mL MES (0.1 M, pH 5.2) was added dropwise to the alginate / EDC / NHS solution and reacted at room temperature for 1 hour. After the reaction, the mixture was dialyzed against 5 L of deionized water (pH 6) and 20 g of NaCl for 3 days (3.5 kDa MWCO dialysis membrane). Then, the mixture was dialyzed against pure deionized water for 4 hours and lyophilized for 3 days to synthesize alginate-catechol (Alginate-C).

The 1H NMR of the synthesized alginate-catechol was measured using AVANCE III 500 (Bruker, USA), and the results are shown in FIG. 2.

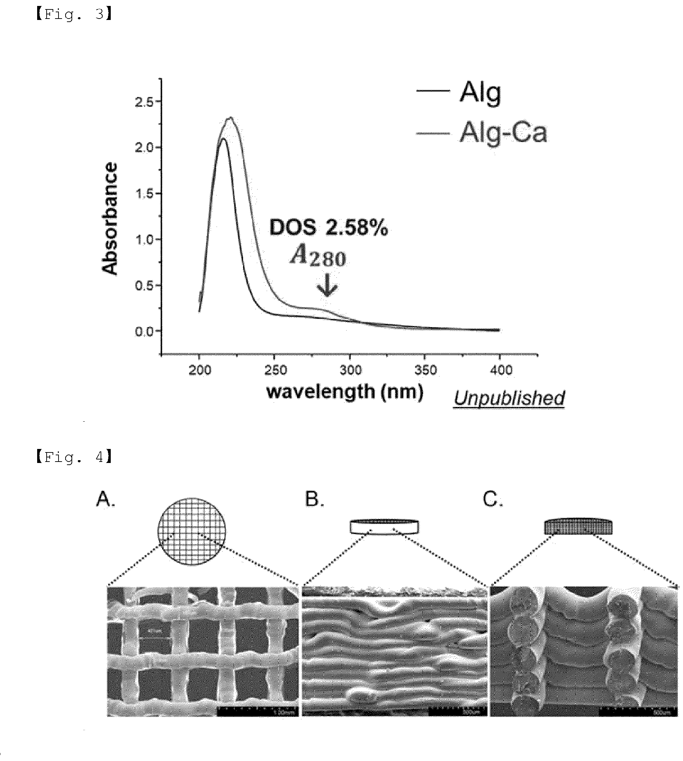

In addition, the degree of substitution (DOS) of catechol in alginate was determined by measuring the absorbance at 280 nm (A280) using a UV-Vis spectrophotometer (UV-1800, Shimadzu, Japan), and a standard curve for DOS calculation was generated using various concentrations of dopamine hydrochloride (0.02-0.1 mg/mL), and the results are shown in FIG. 3.

### Example 2. Manufacture of porous scaffold comprising biodegradable synthetic polymer

### Example 2-1. Manufacture of poly-L-lactic acid (PLLA) porous scaffold (1)

A PLLA porous scaffold with a length (thickness) of 1 mm and a diameter of 5 mm was printed using a 3D printer (Invivo 4D2; ROKIT Healthcare, Seoul, Korea). The PLLA plastic was extruded at a fixed temperature of 200°C, the speed for the scaffold was set to be controlled at 3 mm/s, the discharge pressure was 200-300 kPa, and the manufactured scaffold had a density of 15D (15%).

The manufactured PLLA porous scaffold was coated with a gold thin film (Quorum Q150T ES type, FEI, USA), the morphology was confirmed by SEM (Tescan MIRA3, Brno, Czech Republic) analysis (floor conditions: voltage, 10.0 kV; height, 9.1 mm; and magnification, 50x. section conditions: voltage, 10.0 kV; height, 12.5 mm; magnification, 100x. side-view conditions: voltage, 10.0 kV; height, 9.1 mm; magnification, 70x), and the results are shown in FIG. 4.

### Example 2-2. Manufacture of poly-L-lactic acid (PLLA) porous scaffold (2)

A PLLA porous scaffold was manufactured with a total length of 5 mm, a diameter of 5 mm, and a density of 15D (15%) using the same method as in Example 2-1.

### Example 2-3. Manufacture of poly-L-lactic acid (PLLA) porous scaffold (3)

A PLLA porous scaffold was manufactured with a total length of 5 mm, a diameter of 5 mm, and a density of 20D (20%) using the same method as in Example 2-1.

### Example 2-4. Manufacture of poly-L-lactic acid (PLLA) porous scaffold used (4)

A PLLA porous scaffold was manufactured with a total length of 10 mm, a diameter of 5 mm, and a density of 15D (15%) using the same method as in Example 2-1.

### Example 2-5. Manufacture of poly-L-lactic acid (PLLA) porous scaffold (5)

A PLLA porous scaffold was manufactured with a total length of 10 mm, a diameter of 5 mm, and a density of 20D (20%) using the same method as in Example 2-1.

### Example 2-6. Manufacture of poly-L-lactic acid (PLLA) porous scaffold (6)

A PLLA porous scaffold was printed using a 3D printer (Invivo 4D2; ROKIT Healthcare, Seoul, Korea) to have a length of 15 mm, a diameter of 5 mm (wherein the 1 mm-length portion at each end had a diameter of 6 mm), and a density of 25D (25%). The PLLA plastic was extruded at a fixed temperature of 200°C, the speed for the scaffold was set to be controlled at 3 mm/s, the discharge pressure was 200-300 kPa, and the manufactured scaffold had a density of 25D (25%).

### Example 2-7. Manufacture of poly-L-lactic acid (PLLA) porous scaffold (7)

A PLLA porous scaffold was printed with a total length of 15 mm, a diameter of 5 mm (wherein the 1 mm-length portion at each end had a diameter of 6 mm), and a density of 15D (15%) using the same method as in Example 2-6.

### Example 2-8. Manufacture of poly-L-lactic acid (PLLA) porous scaffold (8)

A PLLA porous scaffold was printed with a total length of 15 mm, a diameter of 5 mm (wherein the 1 mm-length portion at each end had a diameter of 6 mm), and a density of 15D (20%) using the same method as in Example 2-6.

### Example 3. Manufacture of bone-regeneration scaffold

### Example 3-1. Multilayer bone-regeneration scaffold stacked in the order of PLLA porous scaffold / alginate-catechol layer / BMP-2 / collagen / alginate-catechol layer / BMP-2 / collagen

The PLLA porous scaffold manufactured according to Example 2-1 was coated with the alginate-catechol (Alg+Ca) solution (0.5 mg/mL) of Example 1 on a rocker (50 rpm) at room temperature for 24 hours. Next, the PLLA porous scaffold coated with alginate-catechol (Alg+Ca) was coated with BMP-2 by immersion in a rhBMP-2 solution (1 µg/mL, 2 µg/mL) at 4°C for 24 hours. After BMP-2 coating, the scaffold was coated with collagen by immersion in collagen type 1 (3 mg/mL), thereby manufacturing a scaffold stacked in the order of PLLA porous scaffold / alginate-catechol layer / BMP-2 / collagen. In the same order, the scaffold was coated with alginate-catechol (Alg+Ca), rhBMP-2, and collagen once more, and then freeze-dried overnight to manufacture a multilayer bone-regeneration scaffold stacked in the order of PLLA porous scaffold / alginate-catechol layer / BMP-2 / collagen / alginate-catechol layer / BMP-2 / collagen.

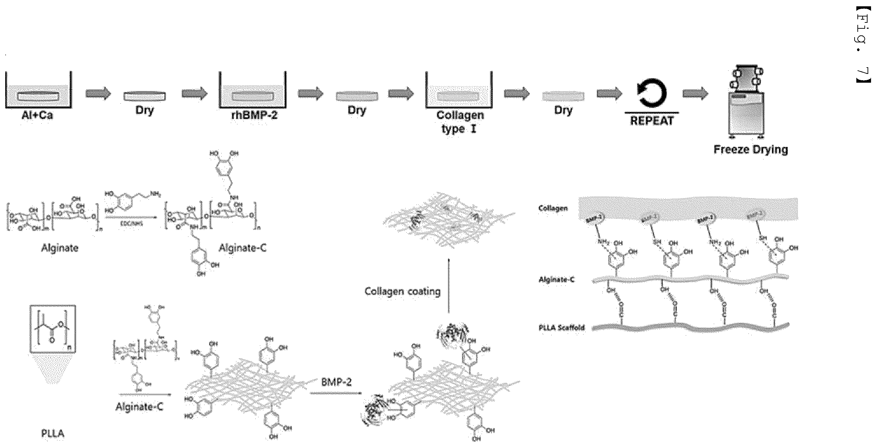

The procedure for sequentially coating alginate-catechol layer / BMP-2 / collagen on PLLA porous scaffold and the binding relationship between the coating layers are as shown in FIG. 7, and the bone-regeneration scaffold stacked form of Example 3-1 is schematically shown in FIG. 8.

### Example 3-2. Multilayer bone-regeneration scaffold stacked in the order of PLLA porous scaffold / alginate-catechol layer / BMP-2 / collagen

The PLLA porous scaffold manufactured according to Example 2-1 was coated with the alginate-catechol (Alg+Ca) solution (0.5 mg/mL) of Example 1 on a rocker (50 rpm) at room temperature for 24 hours. Next, the PLLA porous scaffold coated with alginate-catechol (Alg+Ca) was coated with rhBMP-2 by immersion in a rhBMP-2 solution (1 µg/mL, 2 µg/mL) at 4°C for 24 hours. After BMP-2 coating, the scaffold was coated with collagen by immersion in collagen type 1 (3 mg/mL), thereby manufacturing a multilayer bone-regeneration scaffold stacked in the order of PLLA porous scaffold / alginate-catechol layer / BMP-2 / collagen.

### Example 3-3. Multilayer bone-regeneration scaffold stacked in the order of PLLA porous scaffold / alginate-catechol / collagen / BMP-2 / alginate-catechol / collagen / BMP-2

The PLLA porous scaffold manufactured according to Example 2-1 was coated with the alginate-catechol (Alg+Ca) solution (0.5 mg/mL) of Example 1 on a rocker (50 rpm) at room temperature for 24 hours. Next, the PLLA porous scaffold coated with alginate-catechol (Alg+Ca) was coated with collagen by immersion in collagen type 1 (3 mg/mL) for 1 hour at room temperature. After coating, the scaffold was coated with BMP-2 by immersion in a rhBMP-2 solution (1 µg/mL, 2 µg/mL, 4 µg/mL) at 4°C for 24 hours. Then, the scaffold was sequentially coated with alginate-catechol, collagen, and BMP-2 once more using the same method to manufacture a multilayer bone-regeneration scaffold stacked in the order of PLLA scaffold / alginate-catechol / collagen / BMP-2 / alginate-catechol / collagen / BMP-2.

### Example 3-4. Multilayer bone-regeneration scaffold stacked in the order of PLLA porous scaffold / alginate-catechol / collagen / collagen / BMP-2

The PLLA porous scaffold manufactured according to Example 2-2 or Example 2-4 was coated with the alginate-catechol (Alg+Ca) solution (0.5 mg/mL) of Example 1 on a rocker (50 rpm) at room temperature for 24 hours. The PLLA porous scaffold coated with alginate-catechol (Alg+Ca) was coated with collagen by immersion in collagen type 1 (3 mg/mL) for 2 hours at room temperature. After collagen coating, the scaffold was additionally coated with collagen by immersion in collagen type 1 (3 mg/mL) for 2 hours at room temperature. Then, the scaffold was coated with BMP-2 by immersion in a rhBMP-2 solution (5 µg/mL) at 4°C for 2 hours, and then freeze-dried for 48 hours to manufacture a multilayer bone-regeneration scaffold in which PLLA porous scaffold / alginate-catechol / collagen / collagen / BMP-2 were stacked.

### Example 3-5. Multilayer bone-regeneration scaffold stacked in the order of PLLA porous scaffold / alginate-catechol / collagen / collagen / BMP-2

The PLLA porous scaffold manufactured according to Example 2-3 or Example 2-5 was coated with the alginate-catechol (Alg+Ca) solution (0.5mg/mL) of Example 1 on a rocker (50 rpm) at room temperature for 24 hours. The PLLA porous scaffold coated with alginate-catechol (Alg+Ca) was coated with collagen by immersion in collagen type 1 (3 mg/mL) for 2 hours at room temperature. After collagen coating, the scaffold was additionally coated with collagen by immersion in collagen type 1 (3 mg/mL) for 2 hours at 37°C. Then, the scaffold was coated with BMP-2 by immersion in a rhBMP-2 solution (5 µg/mL) at 4°C for 24 hours, and then freeze-dried for 48 hours to manufacture a multilayer bone-regeneration scaffold in which PLLA porous scaffold / alginate-catechol / collagen / collagen / BMP-2 were stacked.

### Example 3-6. Multilayer bone-regeneration scaffold stacked in the order of PLLA porous scaffold / collagen layer / alginate-catechol layer / BMP-2 / collagen layer / BMP-2 / collagen

The PLLA porous scaffold manufactured according to Example 2-4 was immersed in collagen type 1 (3 mg/mL) for 2 hours at room temperature on a rocker (50 rpm) for 24 hours at room temperature to perform collagen coating on the PLLA porous scaffold. Then, the scaffold was immersed in the alginate-catechol (Alg+Ca) solution (0.5 mg/mL) of Example 1 at room temperature for 3 hours to perform coating, followed by coating with BMP-2 by immersion in the rhBMP-2 solution (5 µg/mL) at 4°C for 24 hours. Next, the scaffold was coated with collagen by immersion in collagen type 1 (3 mg/mL) for 2 hours at room temperature, coated with BMP-2 by immersion in a rhBMP-2 solution (5 µg/mL) at 4°C for 24 hours, coated with collagen by immersion in collagen type 1 (3 mg/mL) for 2 hours at room temperature, and then freeze-dried for 48 hours to manufacture a multilayer bone-regeneration scaffold stacked in the order of PLLA porous scaffold / collagen layer / alginate-catechol layer / BMP-2 / collagen layer / BMP-2 / collagen layer.

### Example 3-7. Multilayer bone-regeneration scaffold stacked in the order of PLLA porous scaffold / collagen layer / alginate-catechol layer / BMP-2 / collagen layer / BMP-2 / collagen

A multilayer bone-regeneration scaffold was manufactured in the same manner as in Example 3-6, except that the PLLA porous scaffold manufactured according to Example 2-2 was used instead of the PLLA porous scaffold manufactured according to Example 2-4.

### Example 3-8. Multilayer bone-regeneration scaffold stacked in the order of PLLA porous scaffold / collagen layer / alginate-catechol layer / BMP-2 / collagen layer / BMP-2 / collagen

A multilayer bone-regeneration scaffold was manufactured in the same manner as in Example 3-6, except that the PLLA porous scaffold manufactured according to Example 2-5 was used instead of the PLLA porous scaffold manufactured according to Example 2-4.

### Example 3-9. Multilayer bone-regeneration scaffold stacked in the order of PLLA porous scaffold / collagen layer / alginate-catechol layer / BMP-2 / collagen layer / BMP-2 / collagen

A multilayer bone-regeneration scaffold was manufactured in the same manner as in Example 3-6, except that the PLLA porous scaffold manufactured according to Example 2-3 was used instead of the PLLA porous scaffold manufactured according to Example 2-4.

### Example 3-10. Multilayer bone-regeneration scaffold stacked in the order of PLLA porous scaffold / alginate-catechol layer / EGCG / collagen / alginate-catechol layer / EGCG / collagen

A multilayer bone-regeneration scaffold was manufactured in the same manner as in Example 3-1, except that an EGCG solution (0.01 µg/mL) was used instead of the rhBMP-2 solution in Example 3-1.

### Example 3-11. Multilayer bone-regeneration scaffold stacked in the order of PLLA porous scaffold / alginate-catechol layer / catechin / collagen / alginate-catechol layer / catechin / collagen

A multilayer bone-regeneration scaffold was manufactured in the same manner as in Example 3-1, except that a catechin solution (0.01 µg/mL) was used instead of the rhBMP-2 solution in Example 3-1.

### Example 4. Manufacture of bone marrow-containing bone-regeneration scaffold

### Example 4-1. Multilayer bone-regeneration scaffold stacked in the order of PLLA porous scaffold / alginate-catechol layer / BMP-2 / collagen / alginate-catechol layer / BMP-2 / collagen, containing bone marrow

A bone marrow-containing bone-regeneration scaffold was manufactured by spraying and impregnating 1 mL or less of bone marrow (BM) into the bone-regeneration scaffold according to Example 3-1 (multilayer bone-regeneration scaffold stacked in the order of PLLA porous scaffold / alginate-catechol layer / BMP-2 / collagen / alginate-catechol layer / BMP-2 / collagen).

The bone marrow may be adsorbed or loaded into the pores of the bone-regeneration scaffold.

### Example 4-2. Multilayer bone-regeneration scaffold stacked in the order of PLLA porous scaffold / alginate-catechol layer / BMP-2 / collagen, containing bone marrow

A bone marrow-containing bone-regeneration scaffold was manufactured by spraying and impregnating 1 mL or less of bone marrow (BM) into the bone-regeneration scaffold according to Example 3-2 (multilayer bone-regeneration scaffold stacked in the order of PLLA porous scaffold / alginate-catechol layer / BMP-2 / collagen).

The bone marrow may be adsorbed or loaded into the pores of the bone-regeneration scaffold.

### Example 5. Manufacture of bone-regeneration scaffold containing epigallocatechin gallate (EGCG)

### Example 5-1. Multilayer bone-regeneration scaffold stacked in the order of PLLA porous scaffold / alginate-catechol layer / EGCG / collagen / alginate-catechol layer / EGCG / collagen, containing epigallocatechin gallate (EGCG)

A bone-regeneration scaffold containing EGCG was manufactured in the same manner as in Example 3-1, except that 100 ug or less of epigallocatechin gallate (EGCG) was used instead of BMP-2.

### Example 5-2. Multilayer bone-regeneration scaffold stacked in the order of PLLA porous scaffold / alginate-catechol layer / EGCG / collagen, containing EGCG

A bone-regeneration scaffold containing EGCG was manufactured in the same manner as in Example 3-2, except that 100 ug or less of epigallocatechin gallate (EGCG) was used instead of BMP-2.

### Example 6. Manufacture of bone-regeneration scaffold containing catechin

### Example 6-1. Multilayer bone-regeneration scaffold stacked in the order of PLLA porous scaffold / alginate-catechol layer / catechin / collagen / alginate-catechol layer / catechin / collagen, containing catechin

A bone-regeneration scaffold containing catechin was manufactured in the same manner as in Example 3-1, except that 100 ug or less of catechin was used instead of BMP-2.

### Example 6-2. Multilayer bone-regeneration scaffold stacked in the order of PLLA porous scaffold / alginate-catechol layer / catechin / collagen, containing catechin

A bone-regeneration scaffold containing catechin was manufactured in the same manner as in Example 3-2, except that 100 ug or less of catechin was used instead of BMP-2.

### <Experimental Examples>

### Experimental Example 1. Morphology analysis of porous scaffold comprising biodegradable synthetic polymer

Morphology analysis of the PLLA porous scaffolds of Examples 2-1 and 2-6 was performed using a scanning electron microscope (SEM). Specifically, the porous scaffold was coated with a gold thin film (Quorum Q150T ES type, FEI, USA), and the morphology was confirmed by SEM (Tescan MIRA3, Brno, Czech Republic) analysis (floor conditions: voltage, 10.0 kV; height, 9.1 mm; and magnification, 50x. section conditions: voltage, 10.0 kV; height, 12.5 mm; magnification, 100x. side-view conditions: voltage, 10.0 kV; height, 9.1 mm; magnification, 70x).

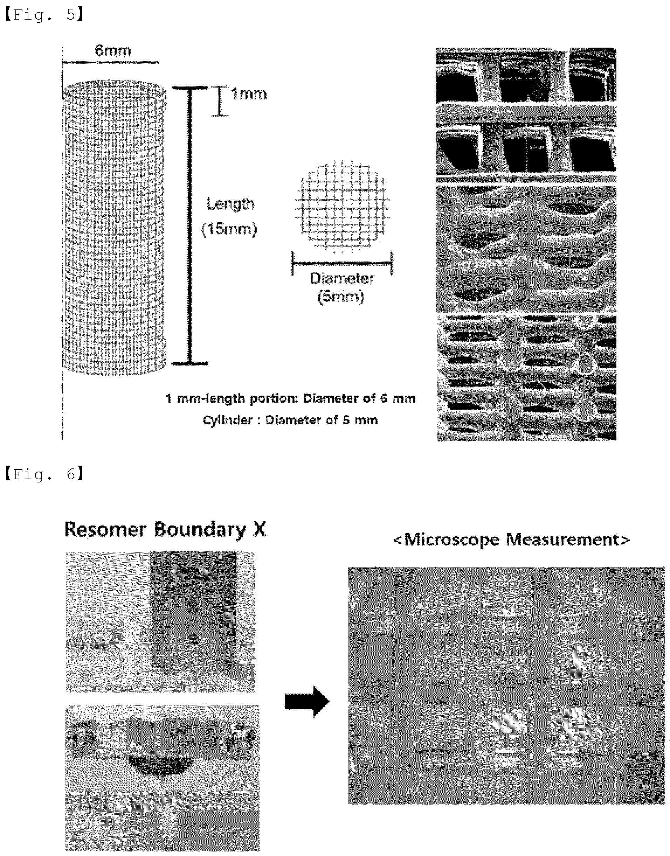

The SEM image analysis results of the porous scaffold of Example 2-1 are as shown in FIG. 4, and the SEM image analysis results of the porous scaffold of Example 2-6 are as shown in FIGS. 5 and 6.

As could be confirmed in FIGS. 5 and 6, the porous scaffold of the present disclosure had pores arranged regularly according to a regularly arranged lattice pattern.

### Experimental Example 2. Analysis of compressive strength and hardness depending on the number of coatings

In order to confirm the change in compressive strength and hardness depending on the number of coatings of the unit comprising alginate-catechol, BMP-2, and collagen, the compressive strength and hardness of Example 3-1 (1 coating), which is the bone-regeneration scaffold in which alginate-catechol, BMP-2, and collagen were sequentially stacked once on the PLLA porous scaffold, Example 3-2 (2 coatings) in which alginate-catechol, BMP-2, and collagen were sequentially stacked twice, and a scaffold in which alginate-catechol, BMP-2, and collagen were sequentially stacked four times (4 coatings) were analyzed.

Specifically, the compressive strength and hardness were measured using a physical property measurement apparatus (SUN RHEO METER) at R/H REAL 20 kg and P/T Press 1 mm/min, and the results are shown in FIG. 9.

As could be confirmed in FIG. 9, when the unit comprising alginate-catechol, BMP-2, and collagen was coated twice (2 coatings) on the PLLA porous scaffold, the compressive strength was 8 times or more greater and the hardness was 2 times or more higher than when coated once (1 coating). In addition, the scaffold coated four times (4 coatings) showed lower compressive strength and hardness than the scaffold coated twice (2 coatings).

Thus, it was proven that the number of times the units comprising alginate-catechol, BMP-2 and collagen were coated on the PLLA porous scaffold affected the compressive strength and hardness, and the suitable number of coatings for the unit comprising alginate-catechol, BMP-2, and collagen was found to be 2 to 3 times.

### Experimental Example 3. Analysis of BMP-2 release behavior

The amount of rhBMP-2 released from the bone-regeneration scaffold was measured by ELISA. PLLA scaffolds were placed in silicone microfuge tubes containing 1 mL of 1x phosphate buffered saline (PBS, pH 7.4) and cultured in a water bath at 37°C. Supernatants were collected daily for 14 days and stored at -20°C. After the end of the experiment, all collected samples were thawed and quantitatively analyzed using a Human BMP-2 ELISA Kit (ab277085, Abcam, UK), and the plates were read spectrophotometrically at 450 nm using a microplate reader (Spectra Max M2, Molecular Devices, USA). Cumulative release was calculated as a percentage of total protein incorporated.

### (1) Results of analysis of release behavior of rhBMP-2 from bone-regeneration scaffold manufactured according to Example 3-2

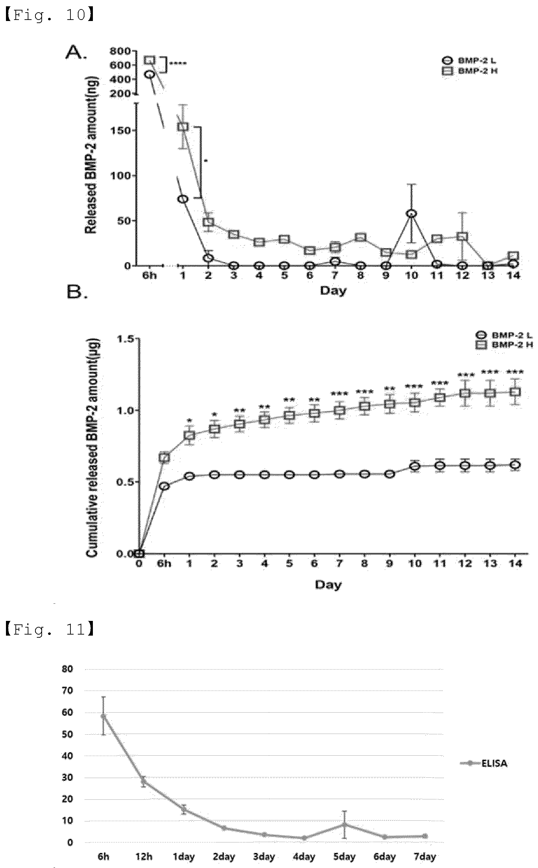

The results of ELISA analysis of the release concentration of rhBMP-2 from the bone-regeneration scaffold according to Example 3-2 are shown in FIG. 10. As could be confirmed in FIG. 10, the amount of BMP-2 released from the bone-regeneration scaffold for 14 days was measured, and on Day 1, approximately 488.25 ± 28.05 ng and 670.61 ± 62.49 ng were released in the L group (1 µg/mL) and the H group (2 µg/mL), respectively (FIG. 10A). The cumulative amount released for 14 days was 617.15 ± 56.44 ng in the L group and 1132.30 ± 125.78 ng in the H group (FIG. 10B). Approximately 10 ng/day of rhBMP-2 was released in the L group, while 35 ng/day of rhBMP-2 was released in the H group.

The results confirmed that the bone-regeneration scaffold of the present disclosure exhibited sustained release characteristics that continuously release rhBMP-2 for 14 days following the initial release.

### (2) Results of analysis of release behavior of rhBMP-1 from bone-regeneration scaffold manufactured according to Example 3-1

The results of analyzing the BMP-2 release concentration from the bone-regeneration scaffold manufactured according to Example 3-1 are shown in FIG. 10. Referring to FIG. 10, the amount of BMP-2 released from the bone-regeneration scaffold for 14 days was measured, and on Day 1, approximately 488.25 ± 28.05 ng and 670.61 ± 62.49 ng were released in the L group (1 µg/mL) and the H group (2 µg/mL), respectively (FIG. 10A). The cumulative amount released for 14 days was 617.15 ± 56.44 ng in the L group and 1132.30 ± 125.78 ng in the H group (FIG. 10B). Approximately 10 ng/day of rhBMP-2 was released in the L group, while 35 ng/day of rhBMP-2 was released in the H group.

The results confirmed that the bone-regeneration scaffold of the present disclosure exhibited sustained release characteristics that continuously release rhBMP-2 for 14 days following the initial release.

### (3) Results of analysis of release behavior of rhBMP-2 from bone-regeneration scaffold manufactured according to Example 3-2

The results of analyzing the BMP-2 release concentration from the bone-regeneration scaffold manufactured according to Example 3-2 are shown in Table 1 and FIG. 11 below (h and day are shown, wherein h means hour).

**[Table 1]**

| Time | ELISA (ppm) |
|---|---|
| 6 h | 58.3 |
| 12 h | 28.0 |
| 1 day | 15.0 |
| 2 day | 6.4 |
| 3 day | 3.4 |
| 4 day | 1.9 |
| 5 day | 8.1 |
| 6 day | 2.4 |
| 7 day | 2.8 |

Referring to Table 1 and FIG. 11, the results of analysis by ELISA after the first two days confirmed that the bone-regeneration scaffold of the present disclosure had sustained-release characteristics that continuously release rhBMP-2 for 7 days following the initial release.

### (4) Results of analysis of release behavior of rhBMP-3 from bone-regeneration scaffold manufactured according to Example 3-3

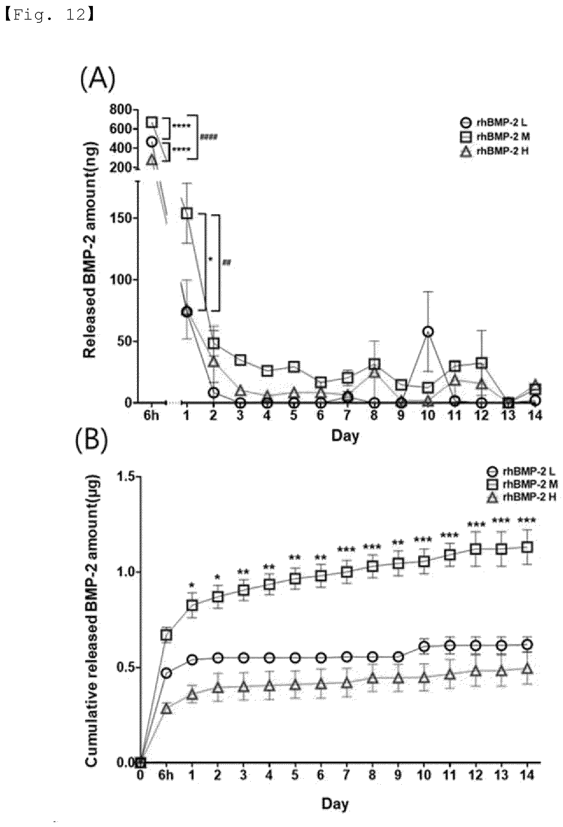

The results of ELISA analysis on the BMP-2 release concentration from the bone-regeneration scaffold manufactured according to Example 3-3 are shown in FIG. 12 (FIG. 12A shows the release amount over time, and FIG. 12B shows the cumulative release amount over time).

Referring to FIG. 12, the release amount was the highest in rhBMP-2 M (2 µg/mL), but was the lowest in rhBMP-2 H (4 µg/mL), as analyzed by ELISA after the first 6 hours. The BMP release amount was low in rhBMP-2 H (4 µg/mL), and the release amount steadily increased over time, showing a sustained release pattern. The absence of burs in rhBMP-2 H was attributed to the interference effect of other coating layers, such as the alginate-catechol layer, gradually collapsed from the last coating layer collagen.

The results confirmed that the bone-regeneration scaffold of the present disclosure exhibited sustained release characteristics that continuously release rhBMP-2 for 14 days following the initial release.

### (5) Results of analysis of release behavior of rhBMP-2 from bone-regeneration scaffold depending on density, coating temperature, and time of porous scaffold

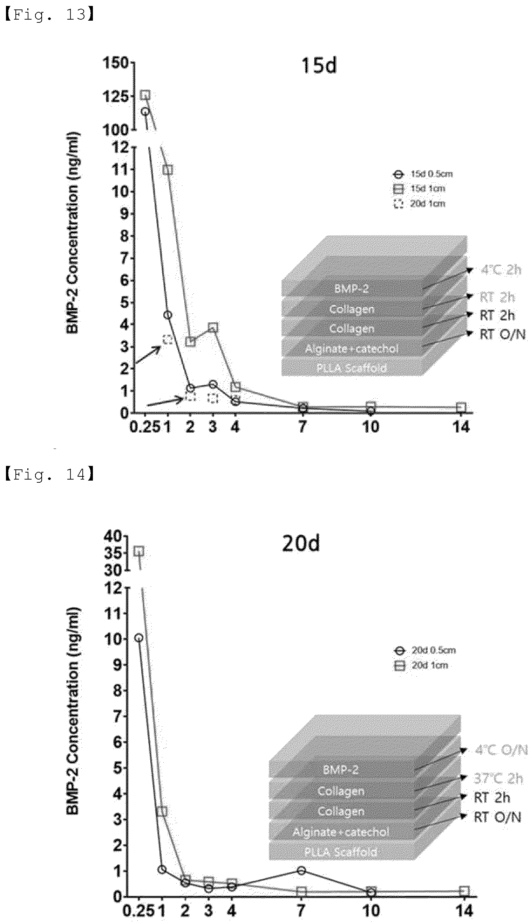

To confirm the BMP-2 release behavior depending on the density, coating temperature, and time of the porous scaffold, the results of ELISA analysis for the BMP-2 release concentration from the bone-regeneration scaffold manufactured according to Examples 3-4 and 3-5 are shown in FIGS. 13 and 14, respectively.

As could be confirmed in FIGS. 13 and 14, there is a difference in the initial release amount according to the scaffold density, and Example 3-4 (15d density group) released the highest concentration (dose) at 6 hours compared to Example 3-5 (20d density group), and Example 3-4 (15d density group) showed a 1-day BMP release concentration 3.5 times higher than that of Example 3-5 (20d density group). Example 3-4 (15d density group) showed a higher release concentration until Day 4 compared to Example 3-5 (20d density group), and the release concentration of Example 3-4 (15d density group) showed a tendency to increase again on Day 3.

The results confirmed that both Examples 3-4 and 3-5 had sustained-release characteristics that continuously release rhBMP-2 for several days following the initial release, and exhibited different release behaviors depending on the density and the coating temperature of the scaffold.

### (6) Results of analysis of release behavior of rhBMP-2 depending on density and length of the porous scaffold

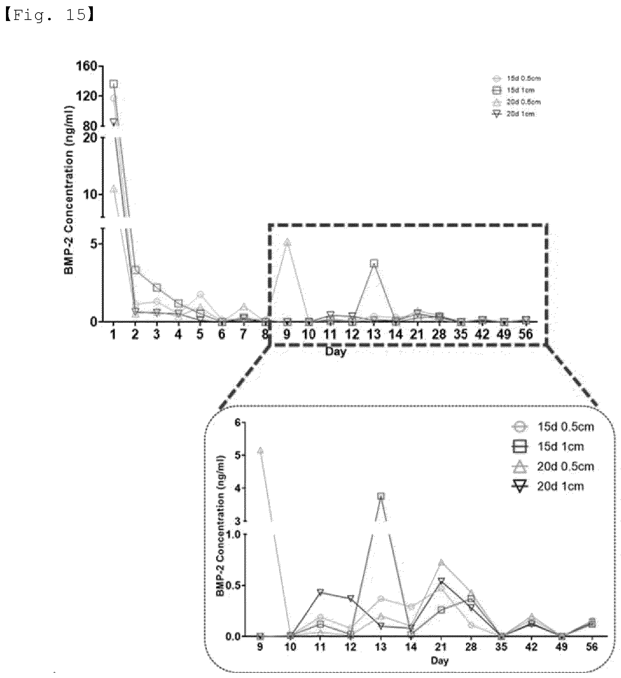

To confirm the BMP-2 release behavior depending on the density and length of the porous scaffold, the results of ELISA analysis for the BMP-2 release concentration from the bone-regeneration scaffolds manufactured according to Examples 3-6 and 3-9 are shown in FIG. 15, respectively.

As could be confirmed in FIG. 15, all of the bone-regeneration scaffolds according to Examples 3-6 to 3-9 were shown to have sustained-release characteristics in which a considerable amount of rhBMP-2 was continuously and regularly released for 56 days. This is because BMP-2 bound with strong binding force was slowly and continuously released until late, and simultaneously, a sufficient amount of BMP-2 required for post-grafting bone regeneration was released as PLLA, a biodegradable synthetic polymer, decomposed. Therefore, the release behavior of BMP-2 is controlled not only by conjugation such as electrostatic bonding and covalent bonding, but also the biodegradability of the biodegradable polymer scaffold.

As a result, the multilayer bone-regeneration scaffolds according to Examples of the present disclosure were shown to have sustained-release characteristics that release BMP-2 at a constant rate over a long period of time.

### Experimental Example 4. Biological Characteristics Analysis (in vitro)

In order to confirm the biological stability and bone regeneration and bone differentiation efficacy of the bone-regeneration scaffolds of the present disclosure, the following *in vitro* experiments were conducted.

### (1) Toxicity and proliferation assay experiments

Cell experiments were performed using the mouse pro-osteoblast MC3T3-E1 cell line or the W-20-17 mouse stromal cell line purchased from the American Type Culture Collection (Manassas, VA, USA; CRL-2593). MC3T3-E1 and W-20-17 cells were cultured in alpha-minimum essential medium (α-MEM) without ascorbic acid (LM008-53; Welgene, Gyeongsan, Korea) and Dulbecco's modified Eagle's medium containing 4500 mg/mL D-glucose and 1500.00 mg/mL sodium bicarbonate (LM001-05; Welgene, Gyeongsan, Korea). *In vitro* cytotoxicity and proliferation tests were performed using scaffold extracts.

Preosteoblast cell line MC3T3-E1 was used to test cytotoxicity and cell proliferation in the presence of PLLA porous scaffolds. MC3T3-E1 cells (7 × 10³ cells/well) were seeded in 96-well plates. The next day, the medium was replaced with α-MEM containing 10% (v/v) release medium (PBS) from the previous BMP-2 release behavior assay. After 48 hours of incubation, the original medium was removed, and 100 µL of fresh medium and 10 µL of CCK-8 solution (Dojindo, Japan) were added to each well, and the plates were incubated at 5% CO₂, 37°C for 100 minutes. The absorbance of each well was measured at 450 nm using a microplate reader (Spectra Max M2, Molecular Devices, USA) for 2 hours.

### (2) Induction of osteoblast differentiation and alkaline phosphatase staining

Alkaline phosphatase (ALP) expression is known to increase under osteocyte differentiation conditions in MC3T3-E1 osteoblastic progenitor cells and the W-20-17 mouse stromal cell line. MC3T3-E1 and W-20-17 cells were seeded in 96-well plates at a density of 1.6 × 10⁴ and 1.3 × 10⁴ cells per well, respectively. The following day, the culture medium was replaced with osteogenic medium (OSM; a-MEM (or DMEM) supplemented with 10 mM β-glycerol phosphate and 50 ug/mL L-ascorbic acid 2-phosphate) and the cells were cultured for 7 days. The medium released from the 24-h culture was added to the OSM medium at 10% (v/v), and the cells treated with PLLA alone and the cells treated with pure BMP-2 (200 ng/mL) were used as negative control (N.C) and positive control (P.C), respectively. After 7 days of induction, the culture medium was aspirated and the cells were washed with saline. The cells were freeze-thawed (-80°C for 30 minutes and then incubated at 37°C for 30 minutes) and lysed in 1% NP-40. The absorbance at 405 nm was measured in a microplate reader using an ALP assay kit (Takara Bio, Shiga, Japan) according to the manufacturer's protocol. The measurement values were normalized to the estimated protein content using the BCA protein assay (Pierce, USA). The ALP activity was expressed as nanomoles of para-nitrophenol/min/mg protein, and ALP staining was performed using an ALP staining kit (BCIP/NBT, Takara Bio, Japan).

### (3) Results of in vitro osteogenic differentiation and bioactivity analysis of released rhBMP-2

The media released from PLLA and PLLA/rhBMP-2 scaffolds did not show any significant differences in cytotoxicity or cell proliferation.

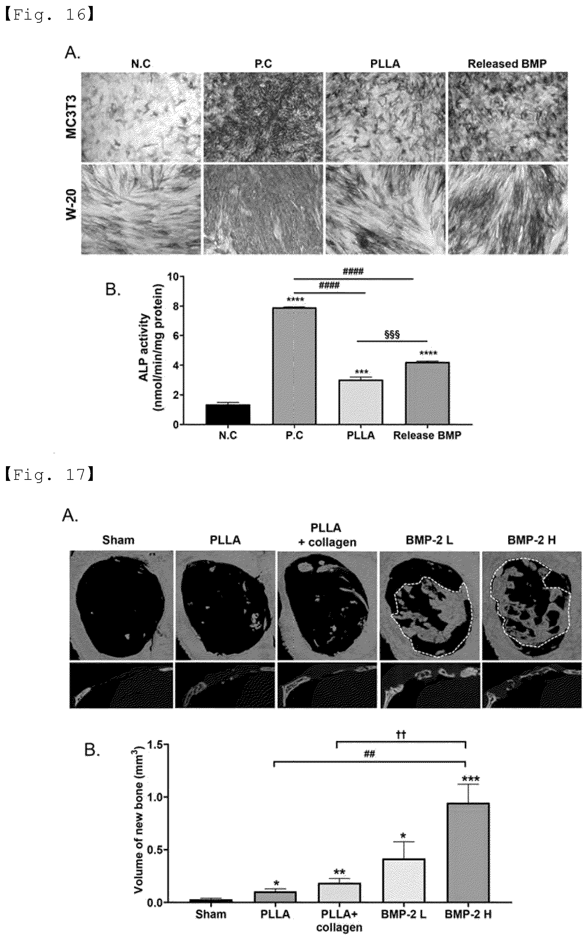

To determine the effect of rhBMP-2-containing media on osteoblast differentiation, MC3T3-E1 and W-20-17 cells were treated with scaffold extracts (PLLA/rhBMP-2, 48.8 ng/mL rhBMP-2) or rhBMP-2 (0.2 µg/mL, positive control). After 7 days of culture, ALP staining was performed to determine the ALP activity, and the results are shown in FIG. 16.

As shown in FIG. 16, both MC3T3-E1 and W-20-17 cells treated with released BMP-2 (Released BMP) or 0.2 µg/mL BMP-2 (P.C) showed increased ALP activity and were positively stained with dark blue (BCIP/NBT reagent), and the ALP activity analysis results also showed that the released rhBMP-2 (Released BMP) maintained its activity. Therefore, it was shown that the treatment with released rhBMP-2 (48.8 ng/mL and 0.2 µg/mL) significantly promoted osteoblast differentiation compared to the treatment with the negative control release medium (PLLA alone, N.C), and the rhBMP-2 released from the scaffold had the property of maintaining its activity.

### Experimental Example 5. Analysis of bone regeneration efficacy using cranial defect mouse animal model (in vivo)

In order to confirm the *in vivo* efficacies (bone formation, bone regeneration) of the bone-regeneration scaffold of the present disclosure, the following experiment was performed using a cranial defect mouse model. In addition, for comparison, a bone regeneration efficacy test was performed using the same process for a control group using PLLA alone and a control group coated with collagen on PLLA.

### (1) Cranial defect mouse model

Animal experiments were performed according to the guidelines approved by Kyungpook National University (Approval No. 2021-0208). Male C57BL/6 mice (7 weeks old; Hyochang Science, Daegu, Korea) were used as a critical-size cranial defect model. Twenty-four C57BL/6 mice were randomly divided into six groups and housed in separate plastic cages and allowed to acclimate to the animal cage conditions for 7 days before surgery. The animals were anesthetized by intraperitoneal (i.p.) injection of sodium pentobarbital (0.3 mL; 1%; 50 mg/kg) on a super clean bench. A sagittal skin incision was made on the scalp, extending from the frontal lobe to the skin flap comprising the occipital bone and periosteum, and a full-thickness circular defect (5 mm in diameter) was then created in the left parietal bone using a 5-mm trephine bur (SAESHIN, Daegu, Korea) and a slow dental handpiece (Saeshin Forte 400s, Daegu, Korea). The trigeminal cranial plate was removed and a disc-shaped scaffold specifically designed to fit the defect site was grafted into the defect site.

The empty calvarial defect was used as a negative control (Sharm), and the experimental groups were designed as Sharm, PLLA alone, PLLA + collagen (collagen-coated PLLA), and the bone-regeneration scaffolds of Example 3-1 (BMP-2 L: 1 µg/mL, BMP-2 H: 2 µg/mL). The animals were housed under conditions of approximately 22 ± 1°C under a 12-h light/dark cycle and were allowed free access to tap water and a standard laboratory diet throughout the experiment.

### (2) Micro-CT imaging

Four weeks after surgery, crania (n = 5) were fixed overnight at room temperature in 10% neutral buffered formalin and then stored in PBS at 4°C until micro-CT. The specimens were scanned using a Skyscan 1275 scanner (Bruker-microCT, Konich, Belgium) in high-resolution mode (X-ray voltage, 55 kV; anode current, 200 mA; aluminum filter, 0.5 mm; isotropic voxel size, 10 nm; exposure time, 100 ms). The scanning was performed using the same calibration parameters and standardized reconstructions were performed using NRecon (Bruker, Kontich, Belgium). The data set was analyzed using CTAn v1.11.10.0 (SkyScan) for newly formed bone and bone fragments matching the defect. For analysis, the region of interest was circled and the upper and lower limits were set to 160 and 60, respectively. The cylindrical volume of interest was defined as 5 mm in diameter and 1 mm in height, which included all new bone formation in the cranial defect. Quantitative CT parameters were analyzed as percentage volume of new bone formation (BV/TV).

### (3) Histological evaluation by hematoxylin and eosin (H&E), Masson's trichrome (MT) staining, and immunohistochemistry (IHC)

Twenty-four samples were were decalcified in 10% ethylenediaminetetraacetic acid (EDTA) contained in paraffin, and sectioned for staining with H&E and MT. Osterix expression in calvaria was detected by IHC using anti-Sp7/osterix (Abcam, ab22552; 1 : 500). A DAB horseradish peroxidase chromogenic kit (Beyotime, Shanghai, China) was used to induce chromogenic reaction. All tissue sections were observed using a Zeiss Axio Scan.Z1 digital slide scanner with a 20x objective lens (Carl Zeiss, Oberkochen, Germany), and areas containing bone regeneration and osteoblasts were analyzed using ImageJ version 1.40 (National Institutes of Health, Bethesda, MD, USA).

### (4) Statistical analysis

Data are expressed as mean ± standard deviation and were analyzed using Student's t-test and one-way analysis of variance (One-Way ANOVA). A-test was used to analyze data between two independent groups, and One-Way ANOVA was performed to analyze data between two or more independent groups. One-Way ANOVA was followed by Tukey's test for post-hoc analysis, and results with P < 0.05 were considered significant.

### (5) Results of bone regeneration analysis

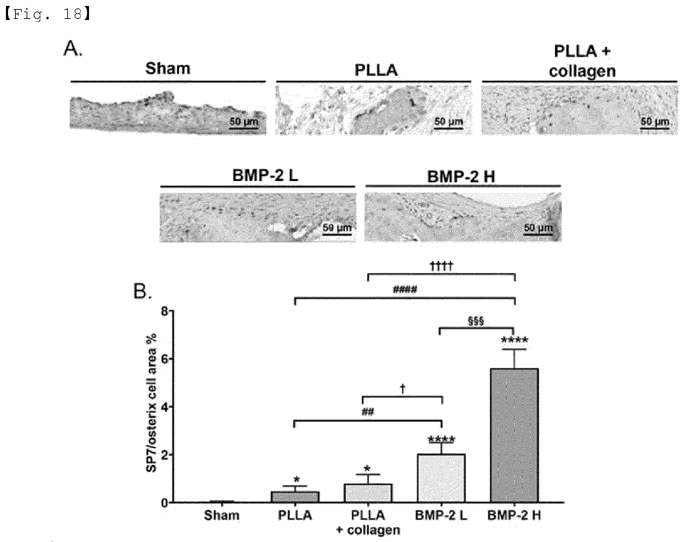

Micro-CT images of samples obtained 4 weeks after cranium grafting are shown in FIG. 17, and the histological evaluation results are shown in FIG. 18.

As could be confirmed in FIG. 17, the Sharm group showed little bone regeneration and bone formation with a bone volume of 0.02 ± 0.008 mm³, while the PLLA alone and PLLA + collagen (PLLA+collagen) groups showed some bone regeneration with a bone volume of 00.10 ± 0.05 mm³ (p = 0.0151) and 0.18 ± 0.10 mm³ (p = 0.0036), respectively, which was slightly higher than that of the Sham group, but no significant difference was found. In contrast, animals in the bone-regeneration scaffolds (rhBMP-2 L and rhBMP-2 H groups) of Example 3-1 showed significantly superior bone regeneration efficacy compared to the other control groups, with bone volumes of 0.41 ± 0.39 mm³ (p = 0.0351) and 1.12 ± 0.26 mm³ (p < 0.0001) in the groups grafted with BMP-2 L and BMP-2 H bone-regeneration scaffolds, respectively.

Further, as could be shown FIG. 18, the osteoblast-positive cell area was 0.24 ± 0.56 mm² in the Sham group, 0.48 ± 0.68 mm², and 0.80 ± 1.38 mm² in the PLLA alone and PLLA + collagen groups, respectively. In contrast, the bone-regeneration scaffold (rhBMP-2 L and H groups) groups in Example 3-1 had a significantly increased osteoblast-positive area of 2.04 ± 1.69 mm² (p = 0.05) and 5.62 ± 2.32 mm² (p = 0.004), respectively, indicating bone regeneration effect.

As a result, the bone-regeneration scaffold of the present disclosure is an excellent bone substitute that releases BMP-2 continuously and constantly in the bone defect site for a long period of time, thereby enhancing bone regeneration and bone differentiation in the bone loss area, and thus has an excellent effect in the treatment of bone diseases.

### Experimental Example 6. Bone regeneration efficacy analysis of bone-regeneration scaffold of the present disclosure using rabbit model (in vivo)

In order to confirm the *in vivo* efficacies (bone formation and bone regeneration) of the bone-regeneration scaffold of the present disclosure, a New Zealand white (NZW) rabbit ulna defect model was utilized, and alginate-catechol coated PLLA scaffold / BMP-2 / collagen (BS), the bone-regeneration scaffold of the present disclosure, was applied to evaluate the bone regeneration effectiveness. One sample per individual was applied to the left forearm ulna of NZW rabbits, and X-ray, computed tomography (CT), and micro-CT evaluations were performed 8 weeks later. Alginate-catechol coated PLLA scaffold / collagen (N = 3) was applied for the control group.

### (1) Rabbit model creation and sample application

① Control group experimental subjects and BS group experimental subjects were moved from the breeding room to the large animal operating room.
② After weight measurement, the experimental subjects were anesthetized by injecting Ketamine (35 mg/kg, IM) / Rompun (5 mg/kg, IM).
③ After confirming the application site (center of the left forearm), the application site and surrounding area were shaved and disinfected with povidone and alcohol.
④ After incising the skin, the muscles were dissected to expose the ulna area.
⑤ An orthopedic saw was used to create a 15 mm-long defect area in the central region of the ulna.
⑥ After the application of the sample, the muscles and skin were sutured.
⑦ After the suture was completed, the experimental subjects were disinfected with povidone and alcohol, followed by dressing, and a splint was used to prevent fractures.
⑧ Upon the subjects' emergence from anesthesia, breathing and reactions were monitored, and subjects exhibiting no abnormalities were identified and transferred to the breeding room.
⑨ Reasons for selecting the application route: It is advantageous for evaluating the effectiveness of long bone regeneration and maintaining a stable state compared to tibia or femur defect models.
⑩ Application method and number of times: The sample was grafted to the left forearm ulna defect (1 ea/animal, single time).

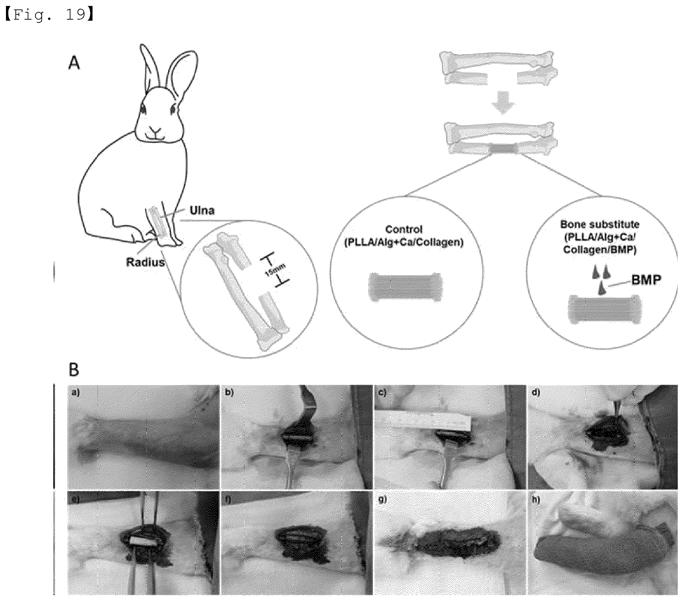

The processes of creating the animal model and applying the sample are shown in FIG. 19.

### (2) Evaluation method

- Observation of general symptoms: In principle, observation was performed once a day. The observation was performed regarding general symptoms and the presence or absence of death, and the subjects with abnormalities were observed for general symptoms by recording the type of symptoms, onset date, and severity of symptoms for each individual as needed.
- Weight measurement: Weight was measured before and at 1-2 week intervals after application of the medical product.
- Imaging evaluation: X-ray imaging was conducted at 0 weeks (OW) and 8 weeks (8W) of application, and CT (SOMATO, SIEMENS) scans were performed at 0 weeks and 8 weeks of application (120 kVp, 0.6 mm slice thickness).

### (3) Results

### ① Body weight

The average body weight before sample application was 2.99 ± 0.10 kg. After 1 week of application, the body weight decreased by 1.43% compared to before application, and after 8 weeks, the body weight was 3.27±0.12 kg, which was a 9.25% increase compared to before application.

### ② Visual evaluation

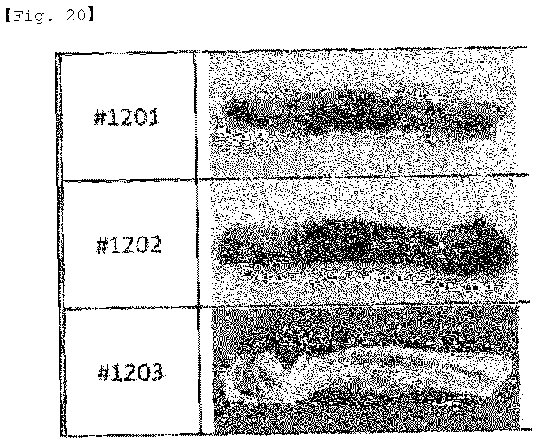

The results of the autopsy of the graft site and visual inspection are shown in FIG. 20. Referring to FIG. 20, there was almost no inflammatory reaction around the graft site.

### ③ X-ray analysis results

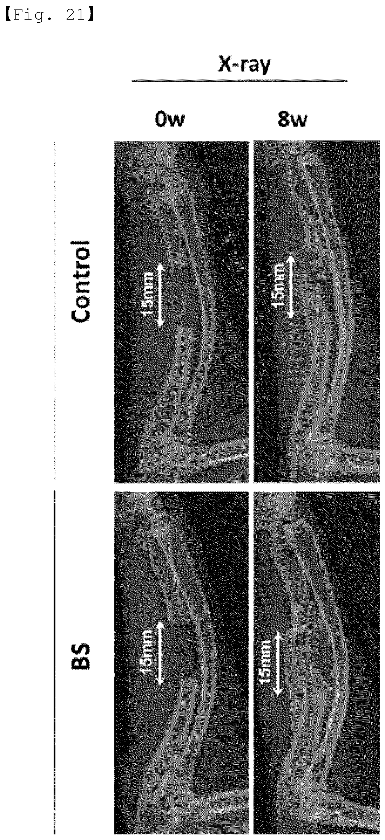

The results of X-ray analysis of the graft site after graft (OW) and 8 weeks (8W) are shown in FIG. 21. As could be confirmed in FIG. 21, the ulna defect area was confirmed immediately after sample application, and the applied sample was not observed through X-ray. In the results at 8 weeks, hard tissue formed along the BS applied sample in the defect area was confirmed.

### ④ CT and Micro-CT analysis results

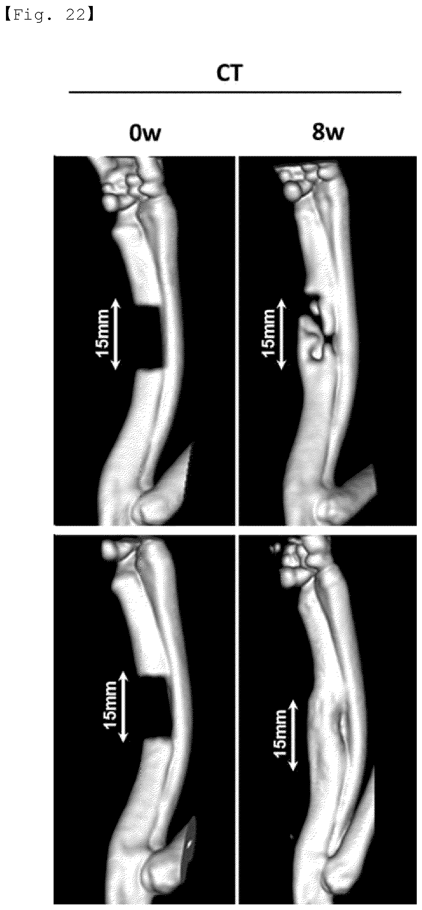

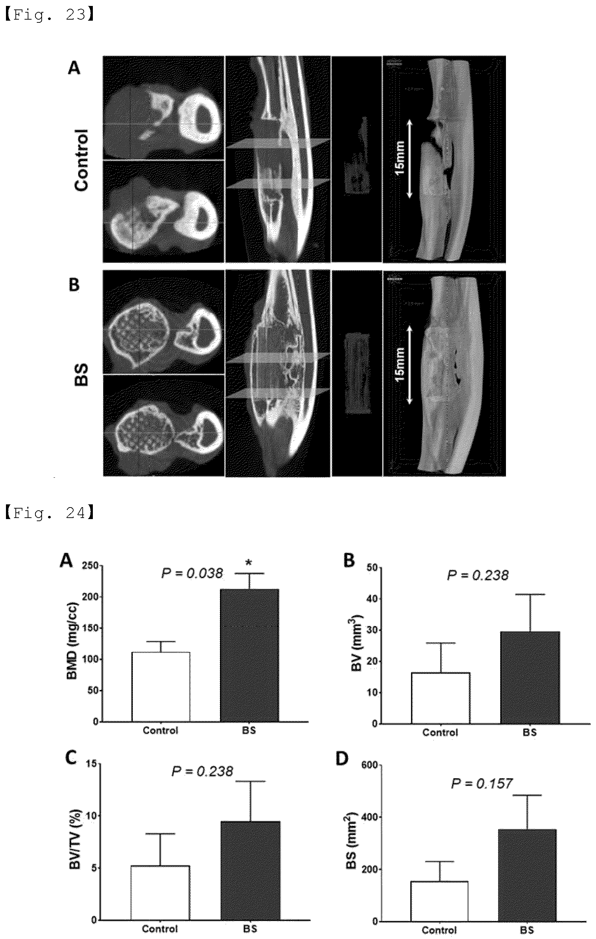

The CT analysis results of the graft site after graft (OW) and 8 weeks (8W) are shown in FIG. 22, and the Micro-CT analysis results image after 8 weeks is shown in FIG. 23. In addition, the results of bone mineral density (BMD) analysis (A), bone volume (BV) (B), bone volume/tissue volume (BV/TV) (C), and bone surface (BS) (D) analysis by Micro-CT are shown in FIG. 24.

As could be confirmed in FIGS. 22 and 23, the control group showed uneven bone regeneration and incomplete bone regeneration. However, the bone-regeneration scaffold-grafted group (BS) of the present disclosure showed that new bone formation was clearly confirmed and the scaffold was connected to the host bone. In addition, the formation of bone along the lattice pattern was clearly identified in the cross-sectional image.

Further, as could be confirmed in FIG. 24, the bone mineral density (BMD) was significantly higher in the BS group (212.9 ± 34.9 mg/cc) than in the control group (111.8 ± 23.4 mg/cc). BV/TV (%) is the mineralized bone volume per unit volume of the sample, and the BV/TV % (9.5 ± 5.4%) of the BS group was significantly higher than that of the control group (5.3 ± 4.3%). These results confirm that graft of the bone-regeneration scaffold according to the present disclosure resulted in significantly superior bone formation.

### Experimental Example 7. Bone regeneration efficacy analysis of bone marrow-containing bone-regeneration scaffold of the present disclosure using rabbit model (in vivo)

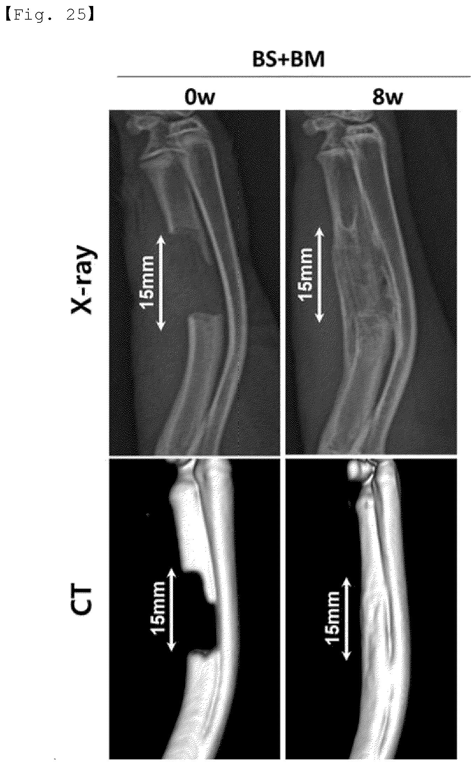

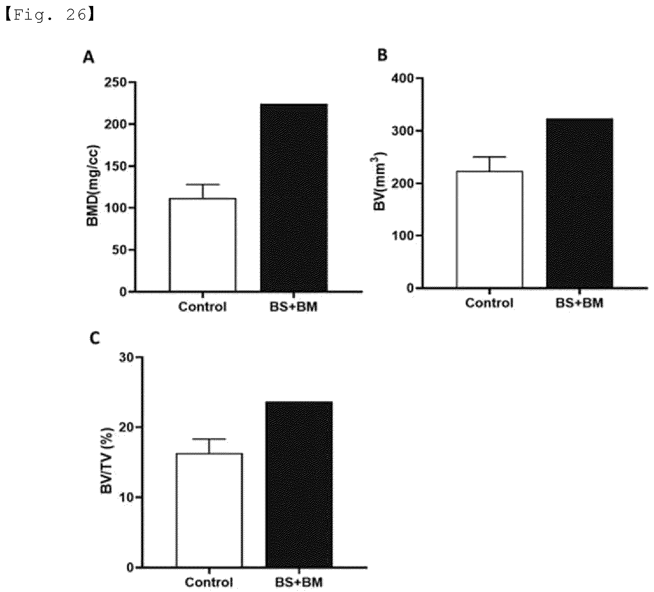

In order to confirm the *in vivo* efficacies (bone formation and bone regeneration) of the bone marrow-containing bone-regeneration scaffold of the present disclosure, a New Zealand white (NZW) rabbit ulna defect model was prepared using the same method as Experimental Example 6, and the bone-regeneration scaffold containing bone marrow of the present disclosure, alginate-catechol coated PLLA scaffold / BMP-2 / collagen (BS+BM) instead of BS, was applied to evaluate the effectiveness of bone regeneration. One sample per individual was applied to the left anterior ulna of NZW rabbits, and X-ray, computed tomography (CT), and micro-CT evaluations were performed 8 weeks later. The results are shown in FIGS. 25 and 26.

As could be confirmed in FIGS. 25 and 26, when the bone-regeneration scaffold containing bone marrow of the present disclosure was grafted, bone regeneration occurred similar to normal bone, and the bone volume and bone density were significantly higher.

### Experimental Example 8. Histological analysis for bone regeneration efficacy confirmation using rabbit model

In order to investigate the biocompatibility of the bone-regeneration scaffold of the present disclosure, histological analysis was performed on samples collected 8 weeks after graft for the animal model experiments performed in Experimental Examples 7 and 8.

Specifically, tissue decalcification was performed on the recovered tissue after sacrificing the animals. The resin was infiltrated into the decalcified tissues and processed to prepare slides, which were then stained with hematoxylin and eosin (H&E) and toluidine blue (TB). The degree of new bone formation and inflammation around the scaffold was histopathologically evaluated using H&E-stained slides, and new bone formation was confirmed with reference to TB-stained slides. To evaluate inflammation and tissue response at the graft site in KMEDI-hub, the semi-quantitative evaluation method (ISO 10993-6) for inflammation and tissue response in Tables 2 and 3 was employed.

The degree of inflammatory cell infiltration observed in the entire graft site and surrounding areas was classified into normal (0), very weak (1+), weak (2+), moderate (3+), and severe (4+), using a semi-quantitative approach. An image analysis device (i-Solution) was used to calculate the new bone area for new bone formation.

**[Table 2]**

| Cell type / Response | Score | | | | |
|---|---|---|---|---|---|
| | 0 | 1 | 2 | 3 | 4 |
| Polymorphonucle ar cells | 0 | Rare, 1-5/phf^{a} | 5-10/phf | Heavy infiltra te | Packed |
| Lymphocytes | 0 | Rare, 1-5/phf | 5-10/phf | Heavy infiltra te | Packed |
| Plasma cells | 0 | Rare, 1-5/phf | 5-10/phf | Heavy infiltra te | Packed |
| Macrophages | 0 | Rare, 1-5/phf | 5-10/phf | Heavy infiltra te | Packed |
| Giant cells | 0 | Rare, 1-2/phf | 3-5/phf | Heavy infiltra te | Sheets |
| Necrosis | 0 | Minimal | Mild | Moderate | Severe |
| ^{a} phf = per high powered (400x) field. | | | | | |

**[Table 3]**

| Response | Score | | | | |
|---|---|---|---|---|---|
| | 0 | 1 | 2 | 3 | 4 |
| Polymorphonu clear cells | 0 | Minimal capillary proliferat ion, focal, 1-3 buds | Groups of 4-7 capillari es with supportin g fibroblas tic structure s | Broad band of capillari es with supportin g structure s | Extensive band of capillari es with supportin g fibroblas tic structure s |
| Lymphocytes | 0 | Narrow band | Moderatel y thick band | Thick band | Extensive band |
| Plasma cells | 0 | Minimal amount of fat associated with fibrosis | Several layers of fat and fibrosis | Elongated and broad accumulat ion of fat cells about the implant site | Extensive fat completel y surroundi ng the implant |

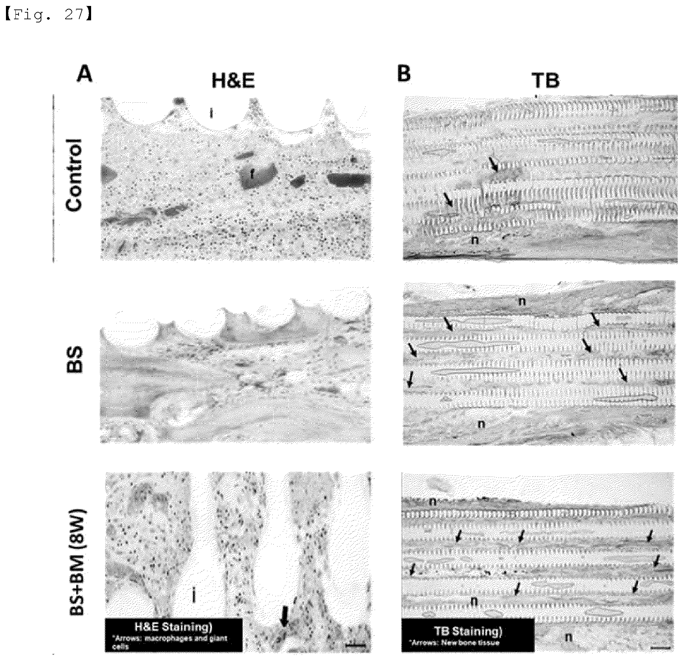

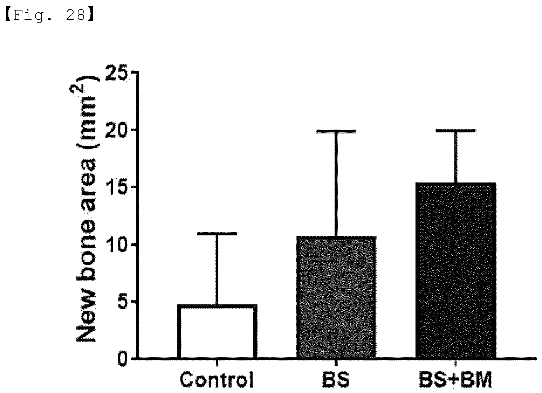

The results of histological analysis using H&E and TB staining are shown in FIG. 27, the results of analyzing the new bone area are shown in FIG. 28, and the results of examining and scoring the histological scores of the number of polymorphonuclear leukocytes (PMN), lymphocytes, plasma cells, macrophages, and giant cells, neovascularization response and fibrosis, bone marrow tissue, and inflammation are shown in Table 4.

**[Table 4]**

| Group | | Control Group | BS | BS+BM |
|---|---|---|---|---|
| Inflammatory cell infiltration, overall | | 2.5 ± 0.7 | 1.3 ± 0.6 | 1.0 ± 0.0 |
| Cell | PMN cells | 1.0 ± 1.4 | 0.0 ± 0.0 | 0.0 ± 0.0 |
| | Lymphocytes | 0.5 ± 0.7 | 1.0 ± 0.0 | 1.0 ± 0.0 |
| | Plasma cells | 1.5 ± 0.7 | 1.0 ± 1.0 | 0.0 ± 0.0 |
| | Macrophages | 2.0 ± 0.0 | 1.7 ± 1.2 | 1.0 ± 0.0 |
| | Giant cells | 0.5 ± 0.7 | 1.7 ± 0.6 | 2.0 ± 0.0 |
| | Necrosis | 0.0 ± 0.0 | 1.0 ± 1.7 | 0.0 ± 0.0 |
| Response | Neovascularizat ion | 1.5 ± 0.7 | 2.0 ± 1.0 | 2.0 ± 0.0 |
| | Fibrosis | 2.5 ± 2.1 | 3.0 ± 0.0 | 2.0 ± 0.0 |
| | Bone marrow tissues | 0.0 ± 0.0 | 2.0 ± 1.7 | 4.0 ± 0.0 |

As could be confirmed in Table 4, the BS group and the BS+BM group showed mild inflammation levels (0 to 2) as determined by analysis of cells involved in inflammation, comprising inflammatory cells and new bone formation, after 8 weeks. In addition, the BS+BM group showed a high bone marrow cell count.

In addition, as shown in FIG. 27, aggregated cells were observed between the 3D printed PLLA layers, and very minimal levels of fibroblasts and inflammatory cells (lymphocytes, macrophages, and giant cells) were identified (FIG. 27A). The results of staining with toluidine blue (pH 7.4) confirmed the dark blue staining (indicated by the arrow in FIG. 7B) indicating mineralized bone, confirming the formation of new bone after grafting of the bone-regeneration scaffold of the present disclosure (FIG. 27B). Furthermore, compared to the control group, the BS group and the BS+BM group observed new bone formation (blue) over a wider area from the scaffold head to the center.

FIG. 28 showed the results of quantitative analysis of bone formation, suggesting that the bone formation in the BS group (10.70 ± 9.18 mm²) and the BS+BM group significantly increased compared to the control group (4.72 ± 6.22 mm²).

As described above, the present disclosure has been described through Examples. Those skilled in the art to which the present disclosure pertains will understand that the present disclosure may be embodied in other specific forms without changing the technical spirit or essential characteristics thereof. Therefore, the above-described Examples should be understood as illustrative in all aspects and not limiting. As the scope of the present disclosure, it should be construed that all changes or modifications derived from the meaning and scope of the claims to be described below and equivalents thereof rather than the above detailed description are included in the scope of the present disclosure.

## Claims

1. A composition for bone regeneration, comprising:
a biodegradable synthetic polymer;
a biocompatible polymer compound comprising catechol groups;
collagen; and
a bioactive substance.

2. The composition for bone regeneration of claim 1, wherein the biodegradable synthetic polymer comprises at least one selected from the group consisting of poly-lactic acid (PLA), poly-L-lactic acid (PLLA), poly-D-lactic acid (PDLA), poly-glycolic acid (PGA), polycaprolactone, and poly-lactic-co-glycolic acid (PLGA).

3. The composition for bone regeneration of claim 1, wherein
the biocompatible polymer compound comprising catechol groups comprises at least one selected from the group consisting of a gelatin-catechol compound, an alginate-catechol compound, a fibrin-catechol compound, a chitosan-catechol compound, and polydopamine.

4. The composition for bone regeneration of claim 1, wherein the biocompatible polymer compound comprising catechol groups is represented by the following Chemical Formula 1: 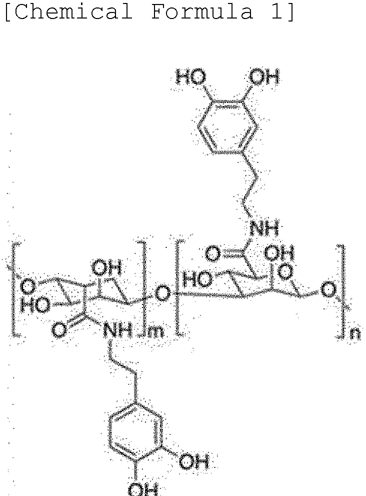 in Chemical Formula 1 above,
m and n are each independently an integer from 1 to 50.

5. The composition for bone regeneration of claim 1, wherein the collagen comprises collagen type I.

6. The composition for bone regeneration of claim 1, wherein the bioactive substance comprises at least one selected from the group consisting of drugs, proteins, peptides, miRNAs, siRNAs, DNAs, plasmid DNAs, small molecules, antibodies, viruses, microorganisms, somatic nuclei, organelles, mitochondria, enzymes, dietary supplements, vitamins, natural products, extracts, growth factors, and cells.

7. The composition for bone regeneration of claim 1, wherein the bioactive substance comprises at least one selected from the group consisting of
bone morphogenetic protein (BMP), platelet derived growth factor (PDGF), transforming growth factor beta (TGF-beta), basic fibroblast growth factor (bFGF), insulin-like growth factor 1 (IGF-1), lactoferrin, bisphosphonate, progenitor cells, bone marrow cells, epithelial cells, fibroblasts, osteoblasts, chondrocytes, cardiomyocytes, myocytes, hepatocytes, human-derived umbilical cord blood cells, mesenchymal stem cells, bone marrow-derived stem cells, periosteum-derived stem cells, vascular endothelial progenitor cells, embryonic stem cells, iPS-derived stem cells, adipose-derived stem cells, placental-derived stem cells, umbilical cord blood-derived stem cells, muscle-derived stem cells, induced pluripotent stem cells, fibroblasts, chondrocytes, osteoblasts, vascular endothelial cells, myoblasts, smooth muscle cells, hepatocytes, neural cells, cardiomyocytes, intervertebral disc cells, catechins and epigallocatechin gallate (EGCG).

8. The composition for bone regeneration of claim 7, wherein the bone morphogenetic protein comprises BMP-1, BMP-2, BMP-3, BMP-4, BMP-5, BMP-6, and BMP-7, or a combination thereof.

9. The composition for bone regeneration of claim 1,
wherein the composition comprises, based on 100 parts by weight of the composition for bone regeneration,
85 to 95 parts by weight of the biodegradable synthetic polymer;
0.5 to 5 parts by weight of the biocompatible polymer compound comprising catechol groups;
4 to 10 parts by weight of the collagen; and
0.05 to 0.5 parts by weight of the bioactive substance.

10. A bone-regeneration scaffold comprising the composition for bone regeneration according to any one of claims 1 to 9.

11. The bone-regeneration scaffold of claim 10, wherein the bone-regeneration scaffold comprises a multilayer structure in which the biodegradable synthetic polymer; the biocompatible polymer compound comprising catechol groups; collagen; and the bioactive substance are alternately and repeatedly stacked in any order.

12. The bone-regeneration scaffold of claim 10, wherein the bone-regeneration scaffold comprises a multilayer structure in which a biocompatible polymer compound layer comprising catechol groups; a collagen layer; and a bioactive substance layer are alternately and repeatedly stacked in any order, on a porous scaffold comprising a biodegradable synthetic polymer.

13. The bone-regeneration scaffold of claim 10, wherein the bone-regeneration scaffold comprises a multilayer structure in which
a biocompatible polymer compound layer comprising catechol groups is stacked on a porous scaffold;
a bioactive substance layer is stacked on the biocompatible polymer compound layer comprising catechol groups; and
a collagen layer is stacked on the bioactive substance layer,
wherein the biocompatible polymer compound layer comprising catechol groups/bioactive substance layer/collagen layer sequentially stacked on the porous scaffold is one unit, and
the units are repeatedly stacked.

14. The bone-regeneration scaffold of claim 13, wherein the unit is repeatedly stacked 2 to 3 times.

15. The bone-regeneration scaffold of claim 12, wherein the porous scaffold is a lattice structure comprising pores.

16. The bone-regeneration scaffold of claim 12, wherein the porous scaffold comprises pores having a diameter of 300 to 600 µm.

17. The bone-regeneration scaffold of claim 12, wherein the porous scaffold has a porosity of 50% to 90%.

18. The bone-regeneration scaffold of claim 12, wherein the porous scaffold has an inter-connectivity of pores of 90% or more.

19. The bone-regeneration scaffold of claim 10, wherein the bone-regeneration scaffold comprises the bioactive substance at a concentration of 0.1 to 5 pg/mL.

20. The bone-regeneration scaffold of claim 12, wherein the porous scaffold has a density of 10 to 30D.

21. A composition for use in the treatment of bone diseases, comprising the composition for bone regeneration according to any one of claims 1 to 9.

22. The composition for use in the treatment of bone diseases of claim 21, wherein the bone disease is selected from the group consisting of growth retardation, osteopenia, bone fragility, osteonecrosis, fracture, osteoporosis caused by excessive osteoclast bone resorption, osteoporotic fracture, diabetic fracture, bone defect, osteogenesis imperfecta, osteomalacia and fractures caused thereby, osteochondral interface defect, bone increase around graft correction, bone growth disorder, bone tumor, nonunion fracture, genetic bone growth defect, osteogenesis imperfecta, bone damage, traumatic joint damage, osteomalacia, rickets, osteitis fibrosa, aplastic bone disease, metabolic bone disease, and renal osteodystrophy.

23. A method for manufacturing a bone-regeneration scaffold, the method comprising:
(1) manufacturing a porous scaffold comprising a biodegradable synthetic polymer;
(2) coating any one of a biocompatible polymer compound comprising catechol groups, collagen, and a bioactive substance on the porous scaffold to form a first coating layer;
(3) coating any one of a biocompatible polymer compound comprising catechol groups, collagen, and a bioactive substance on the porous scaffold to form a second coating layer; and
(4) coating any one of a biocompatible polymer compound comprising catechol groups, collagen, and a bioactive substance on the porous scaffold to form a third coating layer,
wherein the first coating layer, the second coating layer, and the third coating layer are each independently repeated 1 to 3 times.

24. The method of claim 23, wherein the porous scaffold comprising the biodegradable synthetic polymer is manufactured by 3D printing or molding.

25. A sustained-release composition comprising:
a biodegradable synthetic polymer;
a biocompatible polymer compound comprising catechol groups;
collagen; and
a bioactive substance.

26. A sustained-release scaffold comprising the sustained-release composition of claim 25.

27. A sustained-release drug delivery system comprising: a biodegradable synthetic polymer;
a biocompatible polymer compound comprising catechol groups;
collagen; and
a bioactive substance.

28. A method for use in the treatment of bone diseases, comprising: grafting into a subject any one of the composition for bone regeneration of any one of claims 1 to 9; the bone-regeneration scaffold of any one of claims 11 to 20; and the composition for use in the treatment of bone diseases of any one of claims 21 and 22.

29. The method of claim 28, wherein the bone disease is selected from the group consisting of growth retardation, osteopenia, bone fragility, osteonecrosis, fracture, osteoporosis caused by excessive osteoclast bone resorption, osteoporotic fracture, diabetic fracture, bone defect, osteogenesis imperfecta, osteomalacia and fractures caused thereby, osteochondral interface defect, bone increase around graft correction, bone growth disorder, bone tumor, nonunion fracture, genetic bone growth defect, osteogenesis imperfecta, bone damage, traumatic joint damage, osteomalacia, rickets, osteitis fibrosa, aplastic bone disease, metabolic bone disease, and renal osteodystrophy.

30. Use in the prevention or treatment of bone diseases of any one of the composition for bone regeneration of any one of claims 1 to 9; the bone-regeneration scaffold of any one of claims 11 to 20; and the composition for use in the prevention or treatment of bone diseases of any one of claims 21 and 22.

31. The use of claim 30, wherein the bone disease is selected from the group consisting of growth retardation, osteopenia, bone fragility, osteonecrosis, fracture, osteoporosis caused by excessive osteoclast bone resorption, osteoporotic fracture, diabetic fracture, bone defect, osteogenesis imperfecta, osteomalacia and fractures caused thereby, osteochondral interface defect, bone increase around graft correction, bone growth disorder, bone tumor, nonunion fracture, genetic bone growth defect, osteogenesis imperfecta, bone damage, traumatic joint damage, osteomalacia, rickets, osteitis fibrosa, aplastic bone disease, metabolic bone disease, and renal osteodystrophy.
